# EUROPÄISCHE PATENTSCHRIFT

(11) **EP 0 955 369 B1**
(45) Veröffentlichungstag und Bekanntmachung des Hinweises auf die Patenterteilung: **19.04.2006**
(21) Anmeldenummer: 99108502.8
(22) Anmeldetag: 30.04.1999
(51) Int. Cl.: C12N 15/55, C12N 9/16, C12N 1/21, C12N 5/10, C12N 15/79

(54) **Hochaktive alkalische Phosphatase**
High active alkaline phosphatase
Phosphatase alcaline très active

(30) Priorität: 05.05.1998 DE 19819962
(43) Veröffentlichungstag der Anmeldung: 10.11.1999
(73) Patentinhaber: Roche Diagnostics GmbH, 68305 Mannheim (DE)
(72) Erfinder: Hoelke, Werner, Dr., 82377 Penzberg (DE); Müller, Rainer, Dr., 82377 Penzberg (DE); Burtscher, Helmut, Dr., 82392 Habach (DE); Millan, José Luis, Prof., San Diego, CA 92131-3505 (US)

(56) Entgegenhaltungen:
- EP-A- 0 151 320
- DATABASE WPI Week 199445 Derwent Publications Ltd., London, GB; AN 1994-362592 XP002183653 "Recombinant human enteric alkaline phosphatase" (TOSOH CORP), 11. Oktober 1994 (1994-10-11) & DATABASE GSP [Online] TOSOH CORP; AC: AAR63438, 24. Juli 1995 (1995-07-24) "Recombinant human enteric alkaline phosphatase"
- GROTELUESCHEN JEFF ET AL: "Cloning and characterization of the pho-2+ gene encoding a repressible alkaline phosphatase in Neurospora crassa." GENE (AMSTERDAM), Bd. 144, Nr. 1, 1994, Seiten 147-148, XP001018874 ISSN: 0378-1119
- WEISSIG H ET AL: "CLONING AND EXPRESSION OF THE BOVINE INTESTINAL ALKALINE PHOSPHATASE GENE: BIOCHEMICAL CHARACTERIZATION OF THE RECOMBINANT ENZYME" BIOCHEMICAL JOURNAL, PORTLAND PRESS, LONDON, GB, Bd. 290, Nr. 2, 1. März 1993 (1993-03-01), Seiten 503-508, XP002070998 ISSN: 0264-6021
- "Calf Intestine Alkaline Phosphatases " CALZYME LABORATORIES ONLINE CATALOG,CAT NOS. 235B4500 AND 140B4500, 1997, XP002184761
- MANES, T. ET AL.: "Genetic complexity, structure, and characterization of highly active bovine intestinal alkaline phosphatases." JOURNAL OF BIOLOGICAL CHEMISTRY, Bd. 273, Nr. 36, 4. September 1998 (1998-09-04), Seiten 23353-23360, XP001029065
- BESMAN M AND COLEMAN J E: "ISOZYMES OF BOVINE INTESTINAL ALKALINE PHOSPHATASE" JOURNAL OF BIOLOGICAL CHEMISTRY. (MICROFILMS), AMERICAN SOCIETY OF BIOLOGICAL CHEMISTS, BALTIMORE, MD, US, Bd. 260, Nr. 20, 15. September 1985 (1985-09-15), Seiten 11190-11193, XP000606610

## Beschreibung

Die Erfindung betrifft eine DNA kodierend eine eukaryontische hochaktive alkalische Phosphatase mit einer spezifischen Aktivität über 3000 U/mg. Die Erfindung betrifft ferner ein Verfahren zur Herstellung der erfindungsgemäßen DNA, sowie einen Vektor enthaltend die erfindungsgemäße DNA sowie eine Zellinie enthaltend diesen Vektor. Die Erfindung betrifft weiterhin eine rekombinante hochaktive alkalische Phosphatase mit einer spezifischen Aktivität über 3000 U/mg, die durch die erfindungsgemäße DNA kodiert wird.

Alkalische Phosphatasen (AP) sind dimere, zinkhaltige, nichtspezifische Phosphomonoesterasen, die in allen Organismen, von E.coLi bis Säugern vorkommen (McComb et al., 1979). Der Vergleich der Primärstruktur verschiedener alkalischer Phosphatasen ergab einen hohen Homologiegrad (25-30% Homologie zw. E.coli- und Säuger-AP) (Millán, 1988; Harris, 1989).

Im Menschen und höheren Tieren besteht die AP-Familie aus vier Mitgliedern, die auf verschiedenen Genloci codiert sind (Millán, 1988; Harris 1989). Zur alkalischen Phosphatase-Familie zählen die gewebespezifischen APs (Placenta-AP (PLAP), Keimzellen-AP (GCAP) und Darm-AP (IAP)) und die nicht-gewebespezifischen APs (TNAP), die vorwiegend in Leber, Niere und Knochen lokalisiert sind.

Eine entscheidende Eigenschaft der bislang bekannten APs ist die große Variabilität in der katalytischen Aktivität der Säuger-APs, die eine 10-100 fach höhere spezifische Aktivität besitzen als die E.coli AP. Unter den Säuger-APs zeigt die AP aus dem Rinderdarm (bIAP) die höchste spezifische Aktivität. Diese Eigenschaft macht die bIAP attraktiv für biotechnologische Anwendungen wie Enzymkonjugate als diagnostisches Reagenz oder Dephosphorylierung von DNA. Besman und Coleman belegten 1985 die Existenz zweier IAP-Isoenzyme im Rinderdarm, die IAP aus dem Kälberdarm und die IAP aus dem Darm eines erwachsenen Rindes (bIAPs) durch aminoterminale Ansequenzierung der chromatographisch aufgereinigten AP-Fraktionen. Dabei wurde eine klare Unterscheidung am Aminoterminus zwischen der bIAP des erwachsenen Rindes (LVPVEEED) und der bIAP aus dem Kälberdarm (LIPAEEEN) beschrieben. Weissig et al. gelang 1993 durch Klonierung eine genaue biochemische Charakterisierurig einer rekombinanten bIAP (bIAP I) mit einer spezifischen Aktivität von ca. 3000 U/mg und dem N-Terminus LVPVEEED.

Es sind jedoch auch bIAPs aus Kälberdarm mit spezifischen Aktivitäten bis zu 8000 U/mg kommerziell erhältlich (Boehringer Mannheim, Biozyme) Oriental Yeast), die aber bislang nicht weiter charakterisiert waren. Sämtliche Versuche, diese hochaktiven alkalischen Phosphatasen zu klonieren, schlugen fehl. Die Herstellung einer rekombinanten, hochaktiven alkalischen Phosphatase war daher nicht möglich. Um jedoch eine wirtschaftliche Herstellung der hochaktiven alkalischen Phosphatase zu sichern, ist die rekombinante Herstellung zwingend erforderlich.

Demzufolge war es Aufgabe der vorliegenden Erfindung, hochaktive alkalische Phosphatasen rekombinant zur Verfügung zu stellen, die zudem klonierbar sind. Hochaktiv im Sinne der vorliegenden Erfindung bedeutet, daß die erfindungsgemäße alkalische Phosphatase eine um mindestens 10% gesteigerte Aktivität gegenüber vorbekannten alkalischen Phosphatasen aufweist.

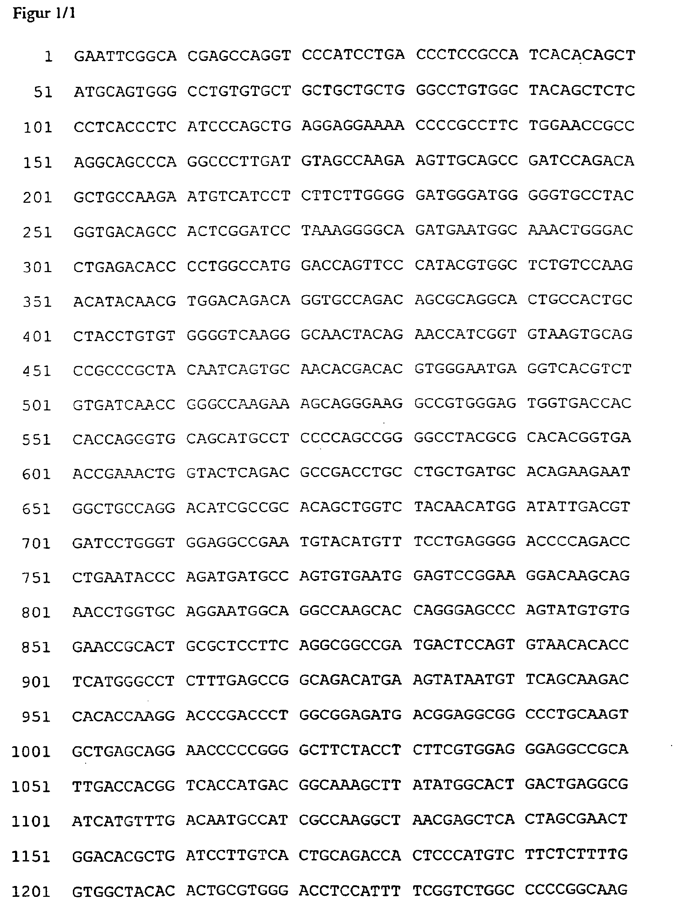

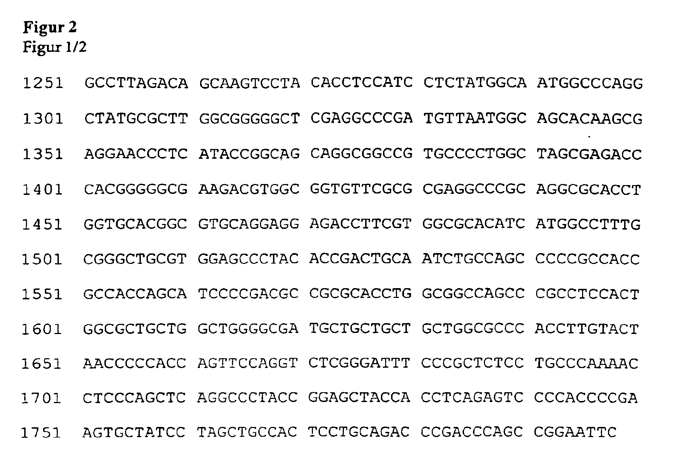

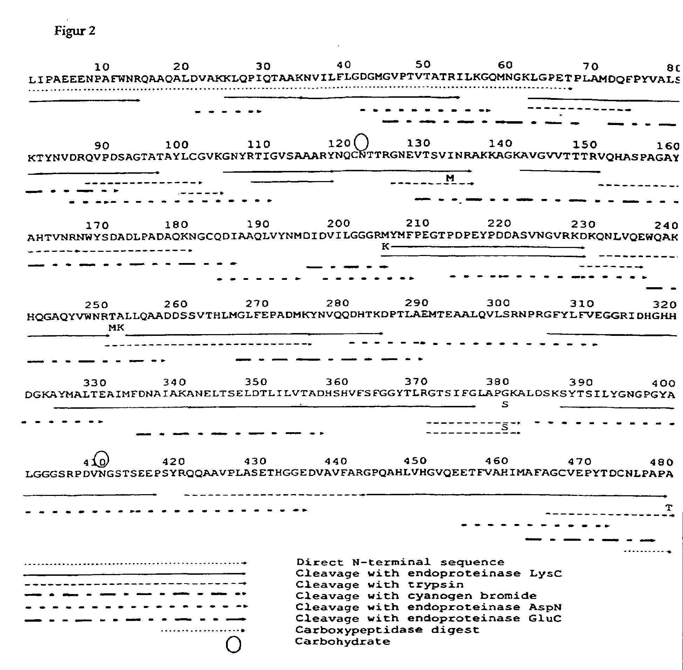

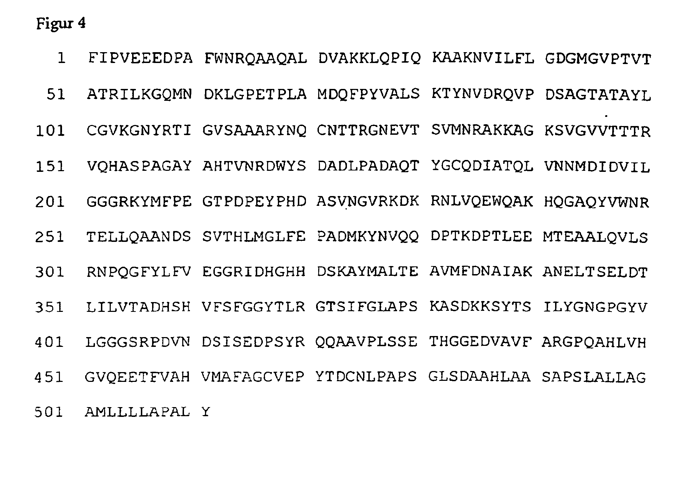

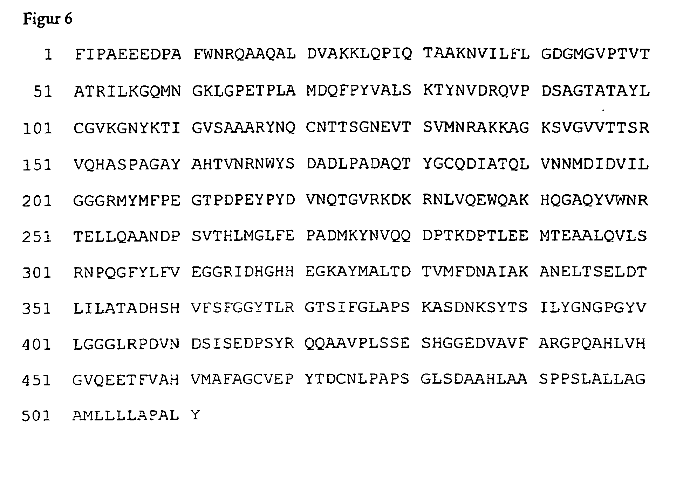

Die Aufgabe wurde erfindungsgemäß gelöst durch die Bereitstellung einer DNA kodierend eine eukaryontische hochaktive alkalische Phosphatase mit einer spezifischen Aktivität über 3000 U/mg, bevorzugt mindestens 3500 U/mg, wobei der Aminosäurerest an der Position 322 kleiner ist als Aspartat. Bevorzugt im Sinne der vorliegenden Erfindung ist eine eukaryontische DNA. Besonders bevorzugt ist eukaryontische cDNA, das heißt eine DNA, die keine Introns mehr enthält. Unter dem Ausdruck "Aminosäurerest kleiner als Aspartat" ist jede Aminosäure, bevorzugt sind natürliche bzw. von diesen abgeleitete Aminosäuren, zu verstehen, die eine kleinere räumliche Ausdehnung als die Struktur der Aminosäure Aspartat aufweist. Bevorzugt ist die erfindungsgemäße DNA, bei der der Aminosäurerest 322 Glycin, Alanin, Threonin, Valin oder Serin ist. Besonders bevorzugt ist eine erfindungsgemäße DNA, bei der der Aminosäurerest 322 Glycin oder Serin ist. Ganz besonders bevorzugt ist, daß der Aminosäurerest 322 Glycin ist. Eine DNA gemäß SEQ ID No.: 1,3 und 5 (Figur 1,3,5) und die zugehörige Aminosäuresequenz gemäß SEQ ID No.: 2,4 und 6 (Figur 2,4,6) sind Teil der vorliegenden Erfindung. Gegenstand der vorliegenden Erfindung sind ebenfalls solche cDNAs, die sich von den obengenannten nur darin unterscheiden, daß der N-Terminus länger oder kürzer ist im Vergleich zu den cDNAs gemäß SEQ ID No.: 2,4 und 6. Entsprechend verändert sich dann die Bezeichnung für die Position 322 gemäß SEQ ID No.: 2,4 und 6. Ist beispielsweise der N-Terminus um x Aminosäuren gegenüber der SEQ ID No.: 2,4 und 6 verlängert oder verkürzt, wird die relevante Position 322 ebenfalls um x Aminosäuren verschoben.

SEQ ID No.: 1 enthält den DNA-Code für die Sequenz des hochaktiven bIAPII-Isoenzyms. Das native Enzym war bekannt, jedoch nicht charakterisiert und nicht klonierbar. Somit ist Gegenstand der vorliegenden Erfindung die Bestimmung der Aminosäuresequenz des hochaktiven bIAP II-Isoenzymes. Zur Sequenzbestimmung wurde eine hoch aufgereinigte Fraktion mit hoher spezifischer Aktivität aus dem Kälberdarm (Boehringer Mannheim) verwendet. Durch Spaltung mit den Endoproteinasen LysC, AspN, GluC, Trypsin sowie chemische Spaltung durch Bromcyan wurden peptide maps der hochaktiven AP erzeugt. Die so erzeugten Peptide wurden getrennt und mittels reversed phase HPLC isoliert. Ober Elektrospray Massenspektroskopie wurde jedes Peptid analysiert und mittels Edman-Abbau sequenziert. Die so erhaltenen Sequenzen wurden mit der veröffentlichten Sequenz der bIAP I (Weissig et al., 1993) verglichen. Wie erwartet besitzt der Aminoterminus von bIAP II die Startsequenz LIPAEEEN, wie von Besman und Coleman beschrieben *U. Biol. Chem.* 260, 11190-11193 (1985)). Die komplette Aminosäuresequenz der bIAP II ist gemäß SEQ ID No.: 2 (Figur 2) dargestellt. Danach weist die bIAP II insgesamt 24 Aminosäureaustausche zu blAP I auf. Die Zahl der Aminosäuren beträgt im isolierten hochaktiven bIAP II Isoenzym 480 Aminosäuren. Die Nukleotidsequenz von 1798 bp (Figur 1) beinhaltet einen kodierenden Bereich von 514 Aminosäuren. Die von Position 481 bis einschließlich 514 möglichen Aminosäuren können dabei in weiten Grenzen variieren.

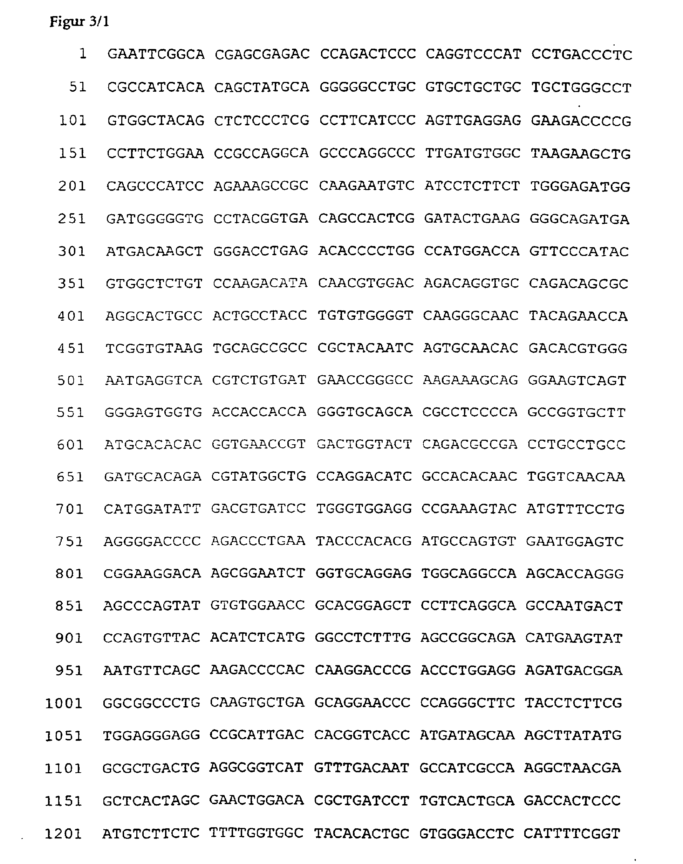

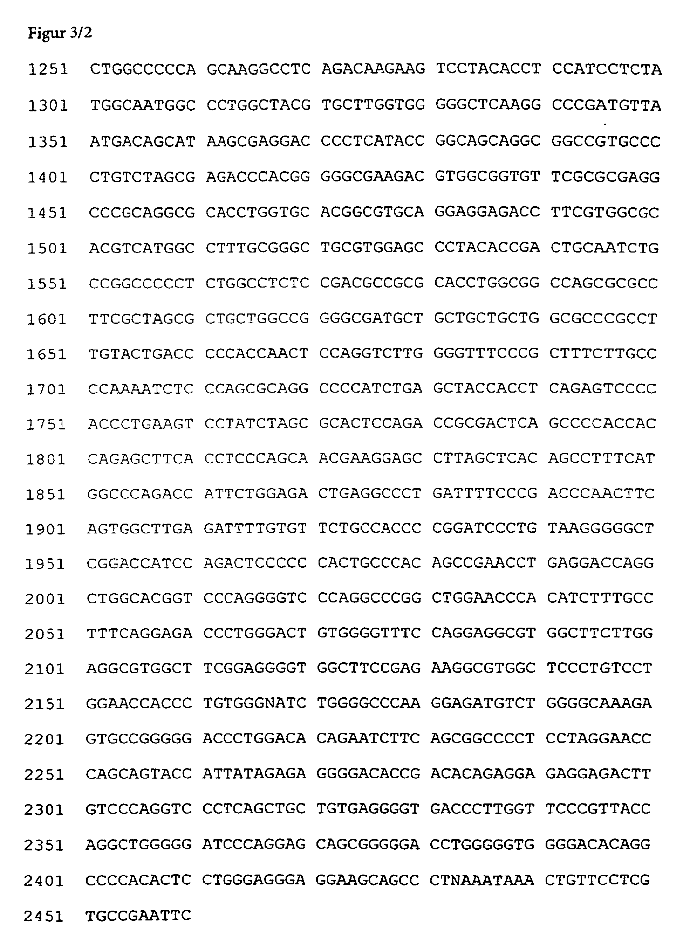

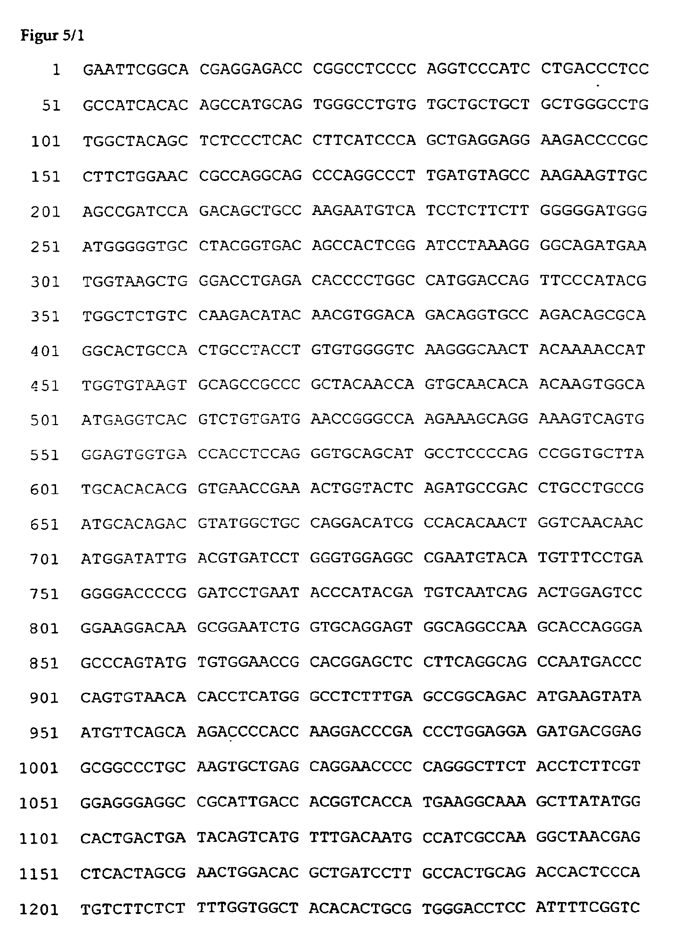

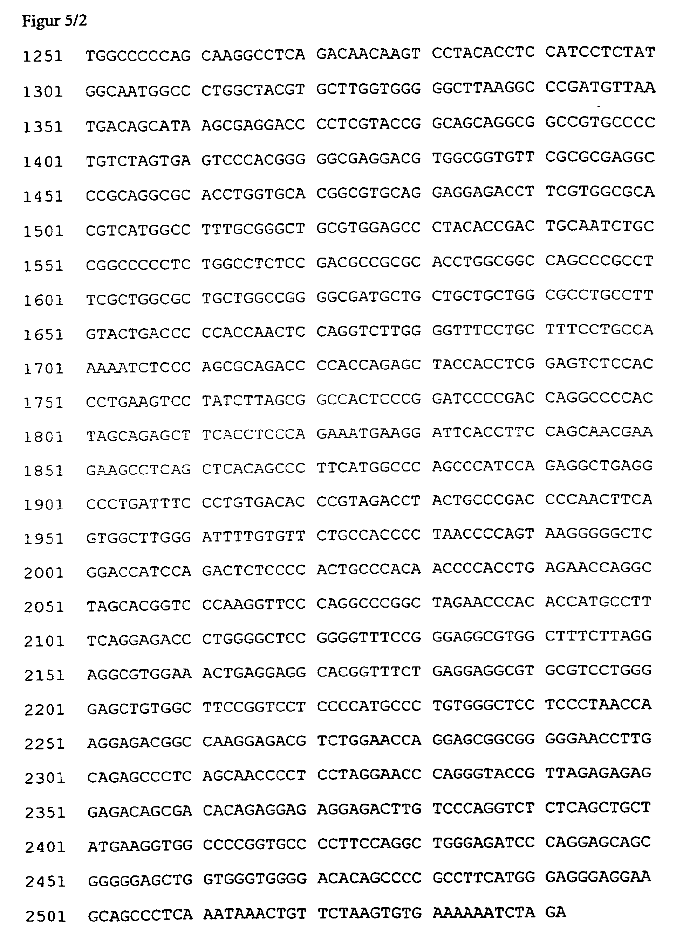

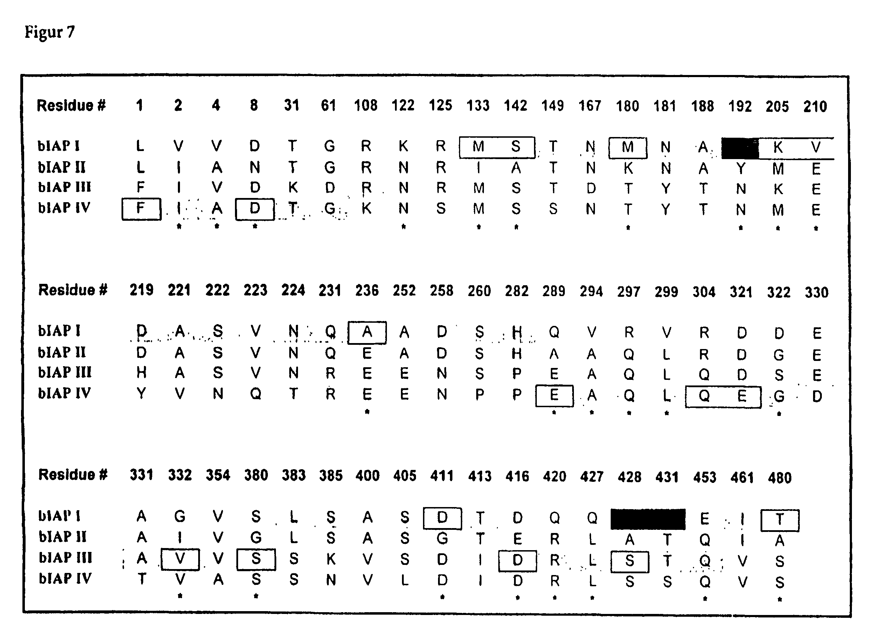

Die vorliegende Erfindung beschreibt des weiteren die Klonierung und vollständige Charakterisierung von zwei neuen, bislang unbekannten bIAPs (bIAP III und bIAP IV). Von RNA-Proben aus verschiedenen Rinderdarmabschnitten wurden Northern Blot Analysen durchgeführt. Von den Proben mit dem stärksten Hybridisierungssignal wurde mit einem Oligo dT-primer (Stratagene, San Diego, CA, USA) eine cDNA-Bank im Vektor IZAP 11 (Stratagene, San Diego, CA, USA) angelegt. Die vollständige Bank (1,0 x 106 rekombinante Klone) wurde mit dem 1075 bp HindIII-Fragment von bIAP I, das einen Bereich von Exon bis VIII des bIAP 1-Gens abdeckt, gescreent. 65 Klone wurden isoliert und sequenziert. Dabei wurden zwei neue bIAPs identifiziert (bIAP III und bIAP IV), die weder zu bIAP I noch zu bIAP II, deren Charakterisierung weiter unten beschrieben ist, vollständig homolog waren. Die Nukleotidsequenzen von bIAP III und IV sind in Figur 3 und 5 abgebildet. Die Sequenzunterschiede der bIAPs I - IV sind in Figur 7 dargestellt. Keine der neuen bIAPs besitzt jedoch den erwarteten N-Terminus LIPAEEEN, sondern neue, bislang noch nicht beschriebene N-Termini (s. Figur 7). Die cDNA der beiden neuen bIAP-Isoenzyme wurde mit entsprechenden Restriktionsenzymen nachgeschnitten und in den CHO-Expressionsvektor pcDNA-3 (z.B. der Fa. Invitrogen, San Diego, CA, USA) durch Ligation insertiert. Die Klone, die die neuen blAP-Isoenzyme enthielten, wurden gemäß dem von Invitrogen beschriebenen Verfahren zur Expression gebracht und die Isoenzyme charakterisiert. In WO 93/18139 wird die Expression eines bIAP-Gens in verschiedenen Wirten beschrieben (CHO-Zellen, E.coli, Baculovirus-System). Gegenstand der vorliegenden Erfindung sind des weiteren die nativen und rekombinanten hochaktiven alkalischen Phosphatasen bIAP III und bIAP IV. Besonders bevorzugt sind die alkalischen Phosphatasen gemäß SEQ ID No.: 4 und 6. CHO-Zellinien enthaltend das bIAP III bzw. bIAP IV Gen wurden hinterlegt bei der DSMZ, Deutsche Sammlung von Mikroorganismen und Zellkulturen GmbH, Mascheroder Weg 1b, D-38124 Braunschweig (DSM ACC 2349, DSM ACC 2350).

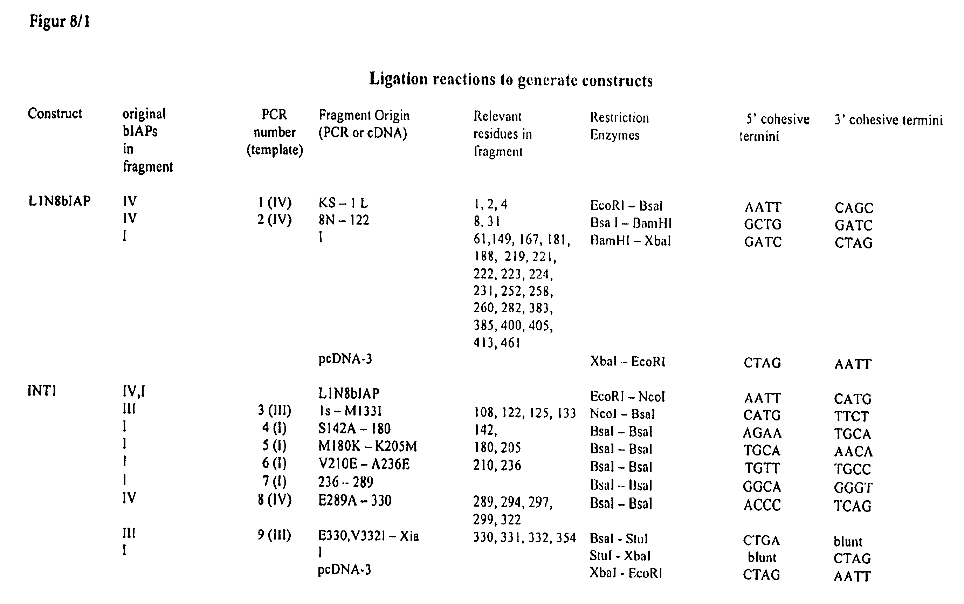

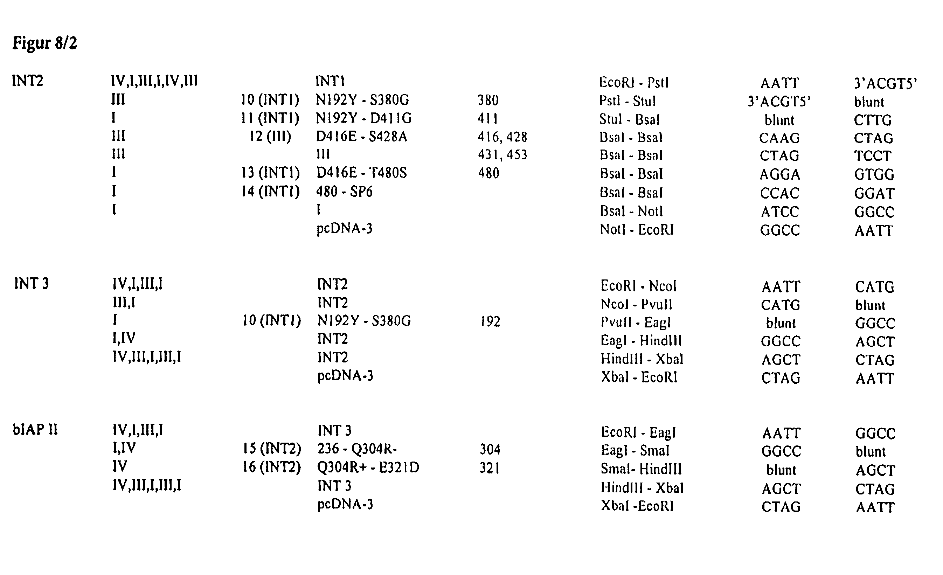

Des weiteren beschreibt die Erfindung die Konstruktion der bIAP II-Sequenz durch Ligation von mutierten- und Wildtyp-Fragmenten von bIAP I, III und IV. Durch diesen Prozeß wurden eine Reihe von intermediären Zwischenprodukten (LIN8, INT 1, INT 2 und INT3) generiert, die für funktionelle Isoenzyme codieren. Zur Konstruktion dieser intermediären Zwischenprodukte wurde jeweils ein Teilstück der zu verändernden bIAP-cDNA mit entsprechenden Restriktionsenzymen herausgeschnitten und durch ein die gewünschten Mutationen enthaltendes Teilstück einer anderen bIAP-cDNA, das durch Verdau mit Restriktionsenzymen kompatible Enden besitzt, ersetzt. Mutationen, die nicht durch Ligation von Teilstücken verschiedener bIAP-cDNAs eingeführt werden konnten, wurden via ortsgerichteter Mutagenese eingebracht. Das mutierte Fragment wurde anschließend mit den entsprechenden Restriktionsenzymen nachgeschnitten und in ein ebenfalls geschnittenes bIAP-cDNA Teilstück mit kompatiblen Enden ligiert (Figur 8). Die so eingeführten Mutationen wurden anschließend via Restriktionsanalyse und Sequenzierung überprüft.

Gegenstand der vorliegenden Erfindung ist somit ein Verfahren zur Herstellung der erfindungsgemäßen DNA, dadurch gekennzeichnet, daß mutierte und Wildtyp-Fragmente der DNA von einer oder mehreren alkalischen Phosphatasen ligiert wurden. Des weiteren ist Gegenstand der vorliegenden Erfindung eine cDNA, die funktionelle Isoenzyme kodiert und die als Zwischenprodukte während des obengenannten erfindungsgemäßen Verfahrens entsteht. Des weiteren ist Gegenstand der vorliegenden Erfinder ein Vektor enthaltend die erfindungsgemäße cDNA.

Des weiteren ist Gegenstand der vorliegenden Erfindung eine Zellinie enthaltend den erfindungsgemäßen Vektor. Geeignete Zellen sind beispielsweise eukaryontische Zellen wie CHO, Pichia, Hansenula oder Saccharomyces cerevisiae und Aspergillus oder prokaryontische Zellen, wie E. coli. Besonders bevorzugt sind E. coli, Hefe- und CHO-Zellen. Geeignete Ausgangsvektoren für E.coli-Stämme sind beispielsweise pTE, pTaq, pPL, pBluescript. Als E. coli-Stämme kommen beispielsweise XL1-Blue, HB 101, RR1 Δ M15, BL 21(DE), MC 1000 etc. in Frage. Geeignete Pichia Vektoren sind beispielsweise pGAPZα und pPICZα (Invitrogen, San Diego, CA, USA). Ein geeigneter Vektor für CHO-Zellinien ist beispielsweise pcDNA-3 (Invitrogen, San Diego, CA, USA). Eine CHO-Zellinie enthaltend das bIAP II Gen wurde hinterlegt bei der DSMZ, Deutsche Sammlung von Mikroorganismen und Zellkulturen GmbH, Mascheroder Weg 1b, D-38124 Braunschweig (DSM ACC 2348).

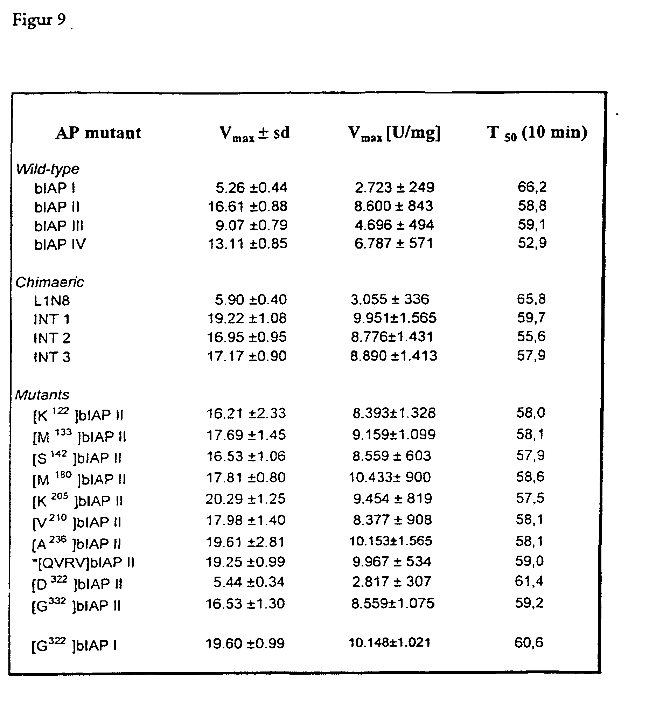

Die kinetische Charakterisierung der rekombinanten bIAP I, II, III und IV-Isoenzyme ergab deutliche Unterschiede hinsichtlich der katalytischen Eigenschaften (Figur 9). bIAP II zeigt beispielsweise eine um über 300% gesteigerte, d.h. über dreifach höhere spezifische Aktivität (ca. 8600 U/mg) als bIAP I (ca. 2700 U/mg). Aber auch bIAP III und bIAP IV zeigen eine etwa 1,8fach (ca. 4700 U/mg) bzw. etwa 2,6fach (>6700 U/mg) höhere Aktivität als bIAP I (Figur 9), was einer prozentualen Steigerung von ca. 170% bzw. 250% entspricht. Zudem war ein beträchtlicher Unterschied der Isoenzyme bezüglich der Stabilität gegenüber Hitze meßbar. bIAP I ist das hitzestabilste Isoenzym, der Tₘ-Wert von bIAP II und III liegt 7°C niedriger und der Tₘ-Wert von bIAP IV 13°C niedriger als bIAP I (Figur 9). Unter dem Tₘ-Wert ist der Temperaturwert zu verstehen, bei dem 50% Restaktivität nach einer Inkubationszeit von 10 Minuten gemessen wird.

Des weiteren beschreibt die Erfindung die Identifikation der Aminosäurereste, die Einfluß auf die spezifische Aktivität der bIAPs besitzen. Dabei waren die intermediären Zwischenprodukte hilfreich. Die Expression der intermediären Chimären L1N8, INT 1, INT 2 und INT 3 ermöglichten es, bereits 11 der 24 Aminosäuren als Effektor für die Aktivitätserhöhung auszuschließen (Figur 7).
- Das L1N8-Mutantenenzym zeigte eine vergleichbare spezifische Aktivität wie bIAP I, demnach sind die hier eingeführten Mutationen V21, V4A und D8N für die Erhöhung der spezifischen Aktivität nicht relevant. Die Bezeichnung V21 bedeutet, daß in Position 2 die Aminosäure Valin gegen Isoleucin ersetzt wird.
- Die INT 1-Mutante besitzt eine vergleichbare spezifische Aktivität mit bIAP II, demzufolge ist dieser Bereich entscheidend.
- Die INT 2-Mutante besitzt eine vergleichbare spezifische Aktivität wie INT 1 und bIAP II, demzufolge können die Mutationen S380G, D411G, D416E, Q420R, Q427L, E453Q und T480A aus INT 2 ebenfalls ausgeschlossen werden.
- Bei der Generierung der INT 3-Mutante konnte ebenfalls keine Änderung der hohen spezifischen Aktivität festgestellt werden, somit ist ein Effekt der Mutation N192Y auszuschließen.

Zur Identifikation, welche der 13 verbliebenen Reste entscheidend für die hohe spezifische Aktivität ist, wurde in der vorliegenden Erfindung die bIAP II-cDNA als Template für Einzelmutationen gegen die entsprechende Aminosäure von bIAP I verwendet. Es wurden die Einzelmutanten N122K, I133M, A142S, K180M, M205K, E210V, E236A, G322D, und I332G sowie eine kombinierte A289Q-A294V-Q297R-L299V-bIAP II-Mutante erstellt (Figur 9).

Überraschenderweise konnte hierbei festgestellt werden, daß hauptsächlich die Mutation G322D in der Lage ist, die hohe spezifische Aktivität von bIAP II (ca. 8600U/mg) um mehr als den Faktor 3 zu senken (2817 U/mg) und somit in die vergleichbar niedrige spezifische Aktivität der bIAP I umzuwandeln.

Zur Verifikation dieses Ergebnisses wurde in der vorliegenden Erfindung die umgekehrte Mutation D322G in bIAP I eingeführt. Überraschenderweise konnte hier der umgekehrte Effekt, nämlich ein Anstieg der spezifischen Aktivität um mehr als Faktor 3 auf 10148 U/mg gemessen werden und somit ein vergleichbarer Wert mit blAP II erzielt werden (Figur 9). Ein Vergleich der Aminosäuresequenzen der relativ höheraktiven bIAP III (ca. 4700 U/mg) und der höheraktiven bIAP IV (>6700 U/mg) bestätigt dieses Ergebnis nochmals. bIAP III besitzt in Position 322 ein Serin, bIAP IV wiederum ein Glycin.

Des weiteren wurden in der vorliegenden Erfindung die erzeugten Mutanten wiederum auf Stabilität gegenüber Hitze untersucht. Demzufolge ist der Unterschied in der Hitzestabilität zwischen blAP I und blAP II auf einen Kombinationseffekt von mehr als einem Austausch zurückzuführen. Sowohl die [G³²²]bIAP I- wie auch die [D³²²]bIAP II-Mutante zeigen Stabilitätswerte, die zwischen denen der bIAP I- und bIAP II-Isoenzyme liegen (Figur 9). Die D322G-Mutation hat einen geringen destabilisierenden Effekt (annähernd 4°C in T₅₀) auf das bIAP I-Isozyrn, während die Substitution G322D in bIAP II eine entsprechende Erhöhung der Stabilität dieses Mutantenenzymes zufolge hat. Jedoch wird die Stabilität gegenüber Hitze der WildtypbIAP I nicht erreicht.

Somit besteht der Gegenstand der vorliegenden Erfindung insbesondere darin, eine hochaktive rekombinante alkalische Phosphatase mit einer Aktivität über 3000 U/mg zur Verfügung zu stellen, die durch eine eukaryontische cDNA kodiert wird. Besonders bevorzugt ist die erfindungsgemäße hochaktive rekombinante alkalische Phosphatase, wobei an der Position 322 ein Glycin, Alanin, Threonin, Valin oder Serin ist. Besonders bevorzugt ist die erfindungsgemäße alkalische Phosphatase, wobei an der Position 322 ein Glycin ist.

Die erfindungsgemäße hochaktive rekombinante alkalische Phosphatase kann bevorzugt zusätzlich an einer oder mehreren der folgenden Positionen eine Mutation aufweisen:

Aminosäurereste in Position 1, 108, 125, 149, 181, 188, 219, 221, 222, 223, 224, 231, 252, 258, 260, 282, 304, 321, 330, 331, 354, 383, 385, 400, 405, 413, 428, 431 und 461, wobei durch die Mutation eine Aktivitätssteigerung bewirkt wird. Gegenstand der vorliegenden Erfindung ist des weiteren ein Verfahren zur Herstellung der erfindungsgemäßen hochaktiven alkalischen Phosphatase. Die erfindungsgemäßen alkalischen Phosphatasen können durch gezielte Mutagenese auch weiter verbessert werden, z.B. hinsichtlich ihrer Thermostabilität.

Die Aktivität der erfindungsgemäßen hochaktiven alkalischen Phosphatase wurde nach E. Mössner et al., Z. Physiol. Chem. 361 (1980), 543-549 bestimmt; mit dem Unterschied, daß der Test nicht, wie in der Publikation beschrieben, bei 25°C, sondern bei 37°C durchgeführt wurde. Die Bestimmung bei 37°C ist die weltweit übliche Temperatur, bei der die Aktivität im Diethanolpuffer (BM Test Method 5426) gemessen wird.

Die Proteinbestimmung der erfindungsgemäßen und bekannten APs erfolgte durch Messen der Absorption der Proteinlösung bei 280 nm gegen Wasser. Die Extinktion einer nieder und hochaktiven AP-Lösung mit einer Konzentration von 1 mg/ml ist bei 280 nm 1,0 (A 280 nm (1 mg/ml) gleich 1).

Die spezifische Aktivität wird durch Verhältnisbildung von Aktivität mit der zugehörigen Proteinmenge ermittelt.

### Erläuterung der Figuren

Figur 1:
   SEQ ID No.: 1 Nukleotidsequenz von bIAP II (1798 bp)
   Start des kodierenden Bereiches für die reife bIAP II in Pos 108, Ende in Pos 1649
Figur 2:
   SEQ ID No.: 2 Aminosäuresequenz von bIAP II (480 Aminosäuren) mit Spaltstellen
Figur 3:
   SEQ ID No.: 3 Nukleotidsequenz von bIAP III (2460 bp)
   Start des kodierenden Bereiches für die reife bIAP III in Pos 123, Ende in Pos 1655
Figur 4:
   SEQ ID No.: 4 Aminosäuresequenz von bIAP III (511 Aminosäuren)
Figur 5:
   SEQ ID No.: 5 Nukleotidsequenz von bIAP IV (2542 bp)
   Start des kodierenden Bereiches für die reife bIAP IV in Pos 122, Ende in Pos 1654
Figur 6:
   SEQ ID No.: 6 Aminosäuresequenz von bIAP IV (511 Aminosäuren)
Figur 7:
   Aminosäureunterschiede zwischen bIAP I, blAP II, bIAP III und bIAP IV Isoenzymen Lediglich die unterschiedlichen Reste werden gezeigt. Mit einem Stern werden solche Positionen identifiziert, die zur individuellen Mutagenese ausgewählt wurden, um die Reste zu identifizieren, die für die erhöhte katalytische Aktivität der bIAP II verantwortlich sind.
Figur 8:
   Ligationsstrategie für die bIAP II - DNA
Figur 9:
   Kinetische Parameter und Hitzestabilität der rekombinanten Wildtyp, chimären und durch ortsgerichtete Mutagenese veränderten Mutanten der bIAP Enzyme.
   * [QVRV]bIAP II ist die Abkürzung für die (Q²⁸⁹, V²⁹⁴, R²⁹⁷, V²⁹⁹]bIAP II Mutante.

Die folgenden Beispiele erläutern die Erfindung weiter:

### Beispiel 1: Klonierung

Eine λgt 11 cDNA Bank präpariert aus Darm von erwachsenen Rindern (Clontech Laboratories, Palo Alto, CA, USA) wurde unter Verwendung eines 1075 bp Hind III Fragments vom 5' Ende der bIAP I cDNA (Weissig et al., 1993) als Sonde gescreent. Klone aus dieser cDNA Bank wurden zum Screenen einer EMBL-3 SP6/T7 genomischen cDNA Bank verwendet, die aus der Leber von erwachsenen Rindern hergestellt war (Clontech Laboratories, Palo Alto, CA, USA). Eine nicht amplifizierte □ZAP II cDNA Bank wurde mittels eines Oligo dT - primers (Stratagene, San Diego, CA, USA) aus mRNA angelegt, die unter Verwendung des Trisolv™ Reagenz aus dem Dünndarm eines erwachsenen Rindes isoliert und mit dem 1075 bp Hind III Fragment der bIAP I cDNA als Sonde gescreent wurde. Die Proben wurden unter Verwenden eines random primed DNA labeling kit radiomarkiert (Boehringer Mannheim). Phagen DNA wurde wie beschrieben für λgt 11 und EMBL-3 SP6/T7 Klone hergestellt (Tsonis & Manes, 1988). Das in vivo Schneiden der □ZAP II Klone wurde nach der Anweisung des Herstellers durchgeführt (Stratagene, San Diego, CA). Genomische Klone wurden mit Southern blot Analyse, wie beschrieben, charakterisiert (Sambrook et al., 1989). EcoRI cDNA Fragmente von □gt 11 Klonen und unterschiedliche Restriktionsfragmente aus Klonen anderer Banken wurden in den KS+ -Vektor (Stratagene, San Diego, CA, USA) subkloniert. Plasmid DNA wurde durch alkalische Lyse hergestellt (Sambrook et al., 1989). Die Sequenzierung erfolgte unter Verwendung von Sequenase gemäß Herstellerprotokoll (Amersham). Die für die Sequenzierung von bIAPs III und IV verwendeten Oligonukleotide sind nachfolgend beschrieben. 1s: SEQ ID No. 7: GCC AAG AAT GTC ATC CTC; 1a: SEQ ID No. 8: GAG GAT GAC ATT CTT GGC; 2s: SEQ ID No. 9: GGT GTA AGT GCA GCC GC; 2a: SEQ ID No. 10: GCG GCT GCA CTT AGA CC; 3s: SEQ ID No. 11: AAT GTA CAT GTT TCC TG; 3a: SEQ ID No. 12: CAG GAA ACA TGT ACA TT; 4s: SEQ ID No. 13: CCA GGG CTT CTA CCT CTT; 4a: SEQ ID No. 14: AAG AGG TAG AAG CCC TGG; 5s: SEQ ID No. 15: ACC AGA GCT ACC ACC TCG; 5a: SEQ ID No. 16: AAG CAG GAA ACC CCA AGA; 6s: SEQ ID No. 17: CTT CAG TGG CTT GGG ATT; 6a: SEQ ID No. 18: AAT CCC AAG CCA CTG AAG. Die Nukleinsäuresequenzen wurden mit dem MacVector Sequenzanalysen-Programmm analysiert (International Biotechnologies, Inc. New Haven, CT, USA).

### Beispiel 2: Bestimmung der Aminosäuresequenz von bIAP II

Ungefähr 500 µg einer gereinigten, hochaktiven (ca. 6000 U/mg) Rinderdarm AP wurde in 450 µl 6M Guanidinhydrochlorid, 0.25 M Tris, 1mM EDTA, pH 8.5 gelöst und anschließend 30 µl Mercaptoethanol hinzugefügt Nach Reduktion in 30 Minuten bei 100°C wurden die Cysteine durch Zugabe von 35 µl Vinylpyridin alkyliert und diese Mischung 45 Minuten bei Raumtemperatur im Dunkeln inkubiert. Das Reaktionsgemisch wurde dann sofort über eine kurze Reversed phase HPLC Aquapore RP300 column entsalzt (30 x 2.1 mm, Applied Biosystents, Weiterstadt). Ein Stufengradient von Acetonitril in 0,1% Trifluoressigsäure wurde verwendet, um gebundene Enzyme zu eluieren. Protein enthaltende Fraktionen wurden bis zur Trockne eingedampft. Um das Enzym zu deglykosilieren, wurden 125 µg AP in 15 µl destilliertem Wasser und 6 µl Inkubationspuffer (250 mM Na₂HPO₄, 50 mM EDTA, pH 7.2) und 15 U EndoF/PNGase gelöst (Boehringer Mannheim, Penzberg). Die Mischung wurde über Nacht bei 37°C gehalten und anschließend zur Spaltung verwendet. Reduzierte und alkylierte AP wurde mit verschiedenen Enzymen gemäß den Anweisungen auf den Datenblättern der einzelnen Enzyme enzymatisch gespalten (Endoproteinase LysC, Endoproteinase AspN, Endoproteinase GluC und Trypsin (Boehringer Mannheim, Penzberg). Cyanbromid Spaltung wurde mit 10% (w/w) CNBr in 70% (v/v) Ameisensäure über 8 Stunden durchgeführt. Nach Lösen mit Wasser wurde die Lösung mit einem SpeedVac Konzentrator (Savant) im Volumen reduziert und für eine Reversed phase HPLC verwendet Der Verdau des C-terminalen tryptischen Peptids wurde über 4 Minuten mit Carboxypeptidase Y (8 ng/µl) durchgeführt und die freigesetzten Peptide mit einer Matrix-unterstützten Laserdesorption/Ionisationsmassenspektrometrie über ein Bruker Reflex III Gerät gemäß Anweisung des Herstellers analysiert. 2,5 Dihydroxybenzoesäure (10 mg/ml) in Acetonitril/Wasser (50/50, v/v) wurde als Matrix verwendet. Peptide aus enzymatischen oder chemischen Spaltungen wurden mit Reversed phase HPLC auf einer LiChrospher C18 selB Säule 125x2 mm (Merck, Darmstadt) unter Einsatz eines 0.1% Trifluoressigsaeure/Acetonitril Lösungsmittelsystems getrennt Die Flußrate betrug 300 µl/min. Der Auslauf wurde mit UV Monitor bei 206 nm detektiert und die Fraktionen manuell gesammelt. Die Massenbestimmung der Peptide-wurde mit einem API III Elektrospray Massenspektrometer (PE-Sciex, Langen) nach den Anweisungen des Herstellers ausgeführt. Die Aminosäuresequenz wurde mit einem 492A Proteinsequenzer (Applied Biosystems, Weiterstadt) gemäß den Herstelleranweisungen ermittelt

### Beispiel 3: Herstellung der bIAP II cDNA und bIAP II Mutagenese

Zur Herstellung einer cDNA, die für bIAP II codiert, wurden Wildtyp Restriktionsfragmente und ortsgerichtet mutagenisierte PCR Fragmente von den cDNAs bIAP I, III und IV miteinander ligiert und die L1N8 (3 Fragmente) and INT 1 (9 Fragmente) cDNA Zwischenkonstrukte geschaffen. INT 1 und bIAP III dienten dann als Vorlage für die ortsgerichtete Mutagenese und Fragmente hieraus wurden zu der kompletten INT 2 (8 Fragmente) cDNA zusammengesetzt. Restriktionsfragmente von INT 2 und ortsgerichtet mutagenisierte Fragmente von INT 2 wurden dann zu der INT 3 (5 Fragmente) cDNA und schließlich zur bIAP II (4 Fragmente) cDNA zusammengesetzt. Die ortsgerichtete Mutagenese wurde nach der Methode von Tomic et al. (1990) durchgeführt, wobei Bsa I (Typ II s) als Restriktionsenzym verwendet wurde, welches in einem Abstand von seiner Erkennungssequenz schneidet (GGTCTCN1/N5). Alle PCR-Produkte wurden sequenziert, um die Abwesenheit von Sekundär-Mutationen zu verifizieren. Alle Konstrukte wurden durch Sequenzierung und Restriktionsverdau bestätigt. Die Sequenz der verwendeten Oligonukleotid-primer zur Amplifikation der ortsgerichteten, mutagenisierten Fragmente sind wie folgt: der Name des primers wird zuerst genannt, gefolgt von der Sequenz (Positionen, die die Mutation anzeigen, sind unterstrichen): KS: SEQ ID No. 19: CGA GGT CGA CGG TAT CG; 1L: SEQ ID No. 20: GCA GGT CTC TCA GCT GGG ATG AGG GTG AGG; 8N: SEQ ID No. 21: GCA GGT CTC AGC TGA GGA GGA AAA CCC CGC; 122: SEQ ID No. 22: GCA GGT CTC TGT TGT GTC GCA CTG GTT; 1s: SEQ ID No. 7: GCC AAG AAT GTC ATC CTC; M133I: SEQ ID No. 23: GGT CTC TTT CTT GGC CCG GTT GAT CAC; S142A: SEQ ID No. 24: GGT CTC AAG AAA GCA GGG AAG GCC GTC; 180: SEQ ID No. 25: GGT CTC GTG CAT CAG CAG GCA GGT CGG C; M180K: SEQ ID No. 26: GGT CTC ATG CAC AGA AGA ATG GCT GCC AG; K205M: SEQ ID No. 27: GGT CTC AAA CAT GTA CAT TCG GCC TCC ACC; V210E: SEQ ID No. 28: GT CTC CAT GTT TCC TGA GGG GAC CCC A; A236E: SEQ ID No. 29: GGT CTC CTG CCA TTC CTG CAC CAG GTT; 236: SEQ ID No. 30: GGT CTC TGG CAG GCC AAG CAC CAG GGA; 289: SEQ ID No. 31: GGT CTC CAG GGT CGG GTC CTT GGT GTG; E289A: SEQ ID No. 32: GGT CTC GAC CCT GGCGGA GAT GAC G; 330: SEQ ID No. 33: GGT CTC CTC AGT CAG TGC CAT ATA; 330E,V332I: SEQ ID No. 34: GGT CTC ACT GAGGCG ATC ATG TTT GAC; XIa: SEQ ID No. 35: TG CAC CAG GTG CGC CTG CGG GCC; N192Y: SEQ ID No. 36: GCC GCA CAG CTG GTC TAC AAC ATG GAT; S380G: SEQ ID No. 37: GCT GTC TAA GGC CTT GCC GGG GGC; N192Y: SEQ ID No. 38: GCC GCA CAG CTG GTC TAC AAC ATG GAT; D411G: SEQ ID No. 39: GGG GGT CTC GCT TGC TGC CAT TAA C; D416E: SEQ ID No. 40: GTT AAT GGT CTC ACA AGC GAG GAA CCC TCG; S428A: SEQ ID No. 41: CCC GTG GGT CTC GCT AGC CAG GGG CAC; D416E: SEQ ID No. 42: GTT AAT GGT CTC ACA AGC GAG GAA CCC TCG; T480S: SEQ ID No. 43: GAT GCT GGT CTC GGT GGA GGG GGC TGG CAG; 480: SEQ ID No. 44: CTG CCA GGT CTC ACC ACC GCC ACC AGC ATC; SP6: SEQ ID No. 45: CAT ACG ATT TAG GTG ACA CTA TAG; 236: SEQ ID No. 46: GGT CTC TGG CAG GCC AAG CAC CAG GGA; Q304R-: SEQ ID No. 47: GTA GAA GCC CCG GGG GTT CCT GCT; Q304+: SEQ ID No. 48: AGC AGG AAC CCC CGG GGC TTC TAC; E321D: SEQ ID No. 49: TGC CAT ATA AGC TTT GCC GTC ATG GTG. Die verschiedenen PCR - Reaktionen sind von 1 - 16 numeriert, die Vorlagen sind entweder Wildtyp cDNAs bIAP I, III oder IV, oder die chimaeren Konstrukte INT 1 oder INT 2. Die Oligonukleotid primer (in Klammern) sind die oben angegebenen. 1. bIAP IV (KS, 1L); 2. bIAP IV (8N, 122); 3.bIAP III (1S, M133I); 4. bIAP I (S142A, 180); 5. bIAP I (M180K, K205M); 6. bIAP I (V210E, A236E); 7. bIAP I (236, 289); 8. bIAP IV (E289A, 330); 9. bIAP III (330E, V332I, XIa); 10. INT1 (N192Y, S380G); 11. INT1 (N192Y, D411G); 12. bIAPIII (D416E, S428A); 13. INT1 (D416E, T480S); 14. INT1 (480, SP6); 15. INT2 (236, Q304R-); 16. INT2 (Q304R+, E321D). Die folgenden Ligationsreaktionen wurden in allen Fällen unter Verwendung des pcDNA-3 (Invitrogen, San Diego, CA) Expressionsvektors durchgeführt Die Fragmente sind gemäß der oben genannten PCR Reaktionsnummer beziffert oder mit dem Namen des Wildtyps oder der chimären cDNA benannt, gefolgt von den Restriktionsenzymen, die zur Bildung des kohäsiven Terminus dieses Fragments benutzt werden. L1N8 = pcDNA-3/EcoRI-XbaI + 1/EcoRI-BsaI + 2/BsaI-BamHI + bIAP I/BamH1-Xbal. INT 1 = pcDNA-3/EcoRI-XbaI +L1N8/EcoRI-NcoI + 3/NcoI-BsaI + 4/BsaI + 5/BsaI + 6/BsaI +7/BsaI + 8/BsaI +9/BsaI-StuI + bIAP I/StuI-XbaI. INT 2 = pcDNA-3/EcoRI-NotI + INT1/EcoRI-PstI + 10/PstI-StuI + 11/StuI-BsaI + 12/BsaI +13/BsaI + 14/BsaI + bIAP I/BsaI-NotI. INT 3 = pcDNA-3/EcoRI-XbaI + INT2/EcoRI-NcoI + INT2/NcoI-PvuII +10/PvuII-EagI + INT2/EagI-HindIII + INT2/HindIII-XbaI- bIAP II = pcDNA-3/EcoRI-XbaI + INT3/EcoRI-EagI + 15/EagI-SmaI + 16/SmaI-HindIII + INT3/HindIII-XbaI.

10 zusätzliche Konstrukte wurden hergestellt, um den Rest (die Reste) zu identifizieren, die für die unterschiedlichen kinetischen Eigenschaften von bIAP I und II verantwortlich sind. Alle Konstrukte wurden in pcDNA-3/EcoRI-XbaI subdoniert. 5 Konstrukte wurden durch den Austausch von Restriktionsfragmenten zwischen L1N8 oder bIAP I (I) und bIAP II (II) hergestellt. L1N8 EcoRI-PmII und (II) PmII-XbaI wurden ligiert, um die [N122K]bIAP II Mutante cDNA herzustellen. (II) EcoRI-BstEII, (I) BstEII-PvuII, (II) PvuII XbaI wurden für die [K180M]bIAP II Mutante cDNA kombiniert (II) EcoRI-Eagl, (I) EagI-BstEII, (II) BstEII-XbaI wurden für die [A289Q, A294V, Q297R, L299V]bIAP II Mutante ligiert. (II) EcoRI-EagI, (II) EagI-BstEII, (I) BstEII-HindIII, (II) HindIII-XbaI fuer die [G322D]bIAP II Mutante. (II) FcoRI-HindIII, (I) HindIII-SacI, (II) Sad-XbaI fuer die [I332G]bIAP II Mutante. 5 andere Positionen erforderten neue ortsgerichtete Mutagenese. Hierfür wurden die folgenden Oligonukleotide verwendet: I133M-: SEQ ID No. 50: GGT CTC TTT CTT GGC CCG GTT CAT CAC; A142S-: SEQ ID No.: 51: TGG TCA CCA CTC CCA CGG ACT TCC CTG; M205K-: SEQ ID No. 52: GGT CTC AAA CAT GTA TTT TCG GCC TCC ACC; E210V+: SEQ ID No. 53: GGT CTC ATG TTT CCT GTG GGG ACC CCA GAC; E236A: SEQ ID No. 54: GGT CTC CTG CCA TGC CTG CAC CAG GTT. Unter Verwendung dieser und der vorher aufgelisteten Oligonukleotide wurden die folgenden 8 PCR Reaktionen (a-h) mit bIAP II als Vorlage durchgeführt: a. 1s, I133M-; b. S142A+, M205K-; c. 1s, A142S-; d. V210E+, 330-; e. E210V+, 330-; f. M180K+, E236A-; g. 236+, 330-; h. S142A, K205M-. Die hieraus entstandenen Produkte wurden subkloniert und sequenziert und dann Fragmente für die folgenden Ligationen isoliert: (II) EcoRI-Ncol, (a) NcoI-Bsal, (b) BsaI, Pvull, (II) Pvull-XbaI fuer I133M. (II) EcoRI-NcoI, (c) NcoI-BstEII, (II) BstEII-PvuII, (II) PvuII-XbaI fuer A142S. (II) EcoRI- BstEII, (b) BstEII-BsaI, (d) BsaI-HindIII, (II) HindIII-XbaI fuer M205K. (II) EcoRI-BstEII, (h) BstEII- BsaI, (e) BsaI-HindIII, (II) HindIII-XbaI fuer E210V. (II) EcoRI-NcoI, (II) NcoI-PvuII, (f) PvuII-BsaI, (g) BsaI-HindIII, (II) HindIII-XbaI fuer E236A.

### Beispiel 4: Produktion und Charakterisierung von rekombinanten Enzymen

Alle cDNAs (bIAP I, bIAP II, bIAP III, bIAP IV und entsprechende Mutanten) wurden in den pcDNA-3 Expressionsvektor kloniert (Invitrogen, San Diego, CA, USA), in Eierstockzellen eines chinesischen Hamsters (CHO Zellen) übertragen und stabile Transfektanten durch Wachsen der Zellen in Gegenwart von 500 µg/ml Geneticin ausgewählt (Gibco, BRL). Recombinante APs wurden aus stabilen übertragenen CHO Zellen wie beschrieben extrahiert (Hoylaerts et al., 1997). Zur Messung von k_{cat} wurden Mikrotiterplatten, die mit 0.1 µg/ml hochaffinem Anti-Rinder AP monoklonalem Antikörper beschichtet waren (Scottish Antibody Production Unit, Lanarkshire, Scotland), mit zunehmenden Enzymkonzentrationen inkubiert Die Aktivität des gebundenen Enzyms wurde als zeitliche Änderung der Absorption bei 405 nm und 20°C nach Hinzufügen von 30 mM p-Nitrophenylphosphat (pNPP) als Substrat in 1.0 M Diethanolamin Puffer (pH 9.8), 1 mM MgCl₂ und 20 µM ZnCl₂ gemessen. Die gebildete p-Nitrophenol Konzentration wurde mit einem Extinktionskoeffizienten von 10,080 liter mol⁻¹ cm⁻¹ errechnet. Handelspräparate mit bekannten spezifischen Aktivitäten (Biozyme Laboratories, 7822 U/mg und Boehringer Mannheim, 3073 U/mg) als auch aufgereinigte bIAP II (8600 U/mg) wurden als Standards verwendet. Die Enzymkonzentration in diesen Lösungen, die den Antikörper absättigten (E°), wurde aus einer Standardkurve Aktivität gegenüber bekannten Enzymkonzentrationen unter identischen Testbedingungen berechnet. Die maximale Substratumsetzung (Vmax) wurde dann durch E° geteilt, um k_{cat}, zu errechnen. Zur Berechnung von Kₘ wurde die Substratkonzentration zwischen 0.25 - 2.0 mM p-Nitrophenylphosphat (pNPP) verändert und die Anfangsreaktionsgeschwindigkeit bei 20°C in einem Zeitraum von 10 Minuten gemessen. Regressionskurven von [pNPP]/v gegen [pNPP] (Hanes Kurven) zur x-Achse ergaben -Kₘ. Teilen der Standardabweichung des berechneten y-Wertes für jeden x-Wert in der Regression durch die Regressionsneigung ergab die Standardabweichung von Kₘ. Vₘₐₓ ± Standardabweichung wurde unter Verwendung der zugehörigen Gleichungen durch Teilen von Kₘ ± Standardabweichung mit dem y-Achsenabschnitt ± Standardabweichung berechnet. Die spezifischen Aktivitäten wurden auf der Basis Antikörper-gesättigte Aktivität im Vergleich zu Biozyme errechnet. Hitzestabilitätskurven wurden durch Inkubation von Extrakten bei 45 - 75 °C erstellt, Zunahme in 5 °C Schritten je 10 Minuten, wie vorher beschrieben (Weissig et al., 1993). Die Aktivität jeder Probe wurde dann wie oben bestimmt und die Restaktivität als verbleibender Prozentanteil, verglichen mit der nicht erhitzten Probe, berechnet. Die Temperatur, bei der 50% Restaktivität übrigbleibt (T₅₀), wurde aus der Restaktivität gegenüber den Temperaturkurven errechnet.

## Patentansprüche

1. DNA gemäß SEQ ID No.: 1 (bIAP II).

2. Verfahren zur Herstellung einer DNA gemäß Anspruch 1, **dadurch gekennzeichnet, daß** mutierte und Wildtyp-Fragmente der cDNA von einer oder mehreren bovinen akalischen Phosphatasen zu einem Gen, das für eine aktive alkalische Phosphatase kodiert, ligiert wurden.

3. Vektor enthaltend eine cDNA gemäß Anspruch 1.

4. Eukaryontische oder prokaryontische Zelle enthaltend einen Vektor gemäß Anspruch 3.

5. Verfahren zur Herstellung einer hochaktiven rekombinanten alkalischen Phosphatase mit einer spezifischen Aktivität über 3000 U/mg, **dadurch gekennzeichnet, daß** eine DNA gemäß Anspruch 1 verwendet wird.

## Claims

1. DNA according to SEQ ID NO.: 1 (bIAP II).

2. Process for producing a DNA as claimed in claim 1, **characterized in that** mutated and wild-type fragments of cDNA of one or several bovine alkaline phosphatases were ligated to form a gene which codes for an active alkaline phosphatase.

3. Vector containing a cDNA as claimed in claim 1.

4. Eukaryotic or prokaryotic cell containing a vector as claimed in claim 3.

5. Process for the production of a highly active recombinant alkaline phosphatase with a specific activity of more than 3000 U/mg, **characterized in that** a DNA as claimed in claim 1 is used.

## Revendications

1. ADN selon SEQ ID N°: 1 (bIAP II).

2. Procédé de préparation d'un ADN selon la revendication 1, **caractérisé en ce que** des fragments mutés et du type sauvage du ADNc ont été soumis à une ligation par une ou plusieurs phosphatases alcalines bovines, en un gène, qui code pour une phosphatase alcaline active.

3. Vecteur contenant un ADNc selon la revendication 1.

4. Cellule eucaryote ou procaryote contenant un vecteur selon la revendication 3.

5. Procédé de préparation d'une phosphatase alcaline recombinée très active ayant une activité spécifique supérieure à 3 000 U/mg, **caractérisé en ce qu'**un ADN selon la revendication 1 est employé.


### SEQUENZPROTOKOLL

(1) ALLGEMEINE ANGABEN:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEFON: 0621/7595482
      (H) TELEFAX: 0621/7594457
   (ii) BEZEICHNUNG DER ERFINDUNG: Hochaktive alkalische Phosphatase
   (iii) ANZAHL DER SEQUENZEN: 54
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 1798 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
   GAATTCGGCA CGAGCCAGGT CCCATCCTGA CCCTCCGCCA TCACACAGCT ATGCAGTGGG 60
   CCTGTGTGCT GCTGCTGCTG GGCCTGTGGC TACAGCTCTC CCTCACCCTC ATCCCAGCTG 120
   AGGAGGAAAA CCCCGCCTTC TGGAACCGCC AGGCAGCCCA GGCCCTTGAT GTAGCCAAGA 180
   AGTTGCAGCC GATCCAGACA GCTGCCAAGA ATGTCATCCT CTTCTTGGGG GATGGGATGG 240
   GGGTGCCTAC GGTGACAGCC ACTCGGATCC TAAAGGGGCA GATGAATGGC AAACTGGGAC 300
   CTGAGACACC CCTGGCCATG GACCAGTTCC CATACGTGGC TCTGTCCAAG ACATACAACG 360
   TGGACAGACA GGTGCCAGAC AGCGCAGGCA CTGCCACTGC CTACCTGTGT GGGGTCAAGG 420
   GCAACTACAG AACCATCGGT GTAAGTGCAG CCGCCCGCTA CAATCAGTGC AACACGACAC 480
   GTGGGAATGA GGTCACGTCT GTGATCAACC GGGCCAAGAA AGCAGGGAAG GCCGTGGGAG 540
   TGGTGACCAC CACCAGGGTG CAGCATGCCT CCCCAGCCGG GGCCTACGCG CACACGGTGA 600
   ACCGAAACTG GTACTCAGAC GCCGACCTGC CTGCTGATGC ACAGAAGAAT GGCTGCCAGG 660
   ACATCGCCGC ACAGCTGGTC TACAACATGG ATATTGACGT GATCCTGGGT GGAGGCCGAA 720
   TGTACATGTT TCCTGAGGGG ACCCCAGACC CTGAATACCC AGATGATGCC AGTGTGAATG 780
   GAGTCCGGAA GGACAAGCAG AACCTGGTGC AGGAATGGCA GGCCAAGCAC CAGGGAGCCC 840
   AGTATGTGTG GAACCGCACT GCGCTCCTTC AGGCGGCCGA TGACTCCAGT GTAACACACC 900
   TCATGGGCCT CTTTGAGCCG GCAGACATGA AGTATAATGT TCAGCAAGAC CACACCAAGG 960
   ACCCGACCCT GGCGGAGATG ACGGAGGCGG CCCTGCAAGT GCTGAGCAGG AACCCCCGGG 1020
   GCTTCTACCT CTTCGTGGAG GGAGGCCGCA TTGACCACGG TCACCATGAC GGCAAAGCTT 1080
   ATATGGCACT GACTGAGGCG ATCATGTTTG ACAATGCCAT CGCCAAGGCT AACGAGCTCA 1140
   CTAGCGAACT GGACACGCTG ATCCTTGTCA CTGCAGACCA CTCCCATGTC TTCTCTTTTG 1200
   GTGGCTACAC ACTGCGTGGG ACCTCCATTT TCGGTCTGGC CCCCGGCAAG GCCTTAGACA 1260
   GCAAGTCCTA CACCTCCATC CTCTATGGCA ATGGCCCAGG CTATGCGCTT GGCGGGGGCT 1320
   CGAGGCCCGA TGTTAATGGC AGCACAAGCG AGGAACCCTC ATACCGGCAG CAGGCGGCCG 1380
   TGCCCCTGGC TAGCGAGACC CACGGGGGCG AAGACGTGGC GGTGTTCGCG CGAGGCCCGC 1440
   AGGCGCACCT GGTGCACGGC GTGCAGGAGG AGACCTTCGT GGCGCACATC ATGGCCTTTG 1500
   CGGGCTGCGT GGAGCCCTAC ACCGACTGCA ATCTGCCAGC CCCCGCCACC GCCACCAGCA 1560
   TCCCCGACGC CGCGCACCTG GCGGCCAGCC CGCCTCCACT GGCGCTGCTG GCTGGGGCGA 1620
   TGCTGCTGCT GCTGGCGCCC ACCTTGTACT AACCCCCACC AGTTCCAGGT CTCGGGATTT 1680
   CCCGCTCTCC TGCCCAAAAC CTCCCAGCTC AGGCCCTACC GGAGCTACCA CCTCAGAGTC 1740
   CCCACCCCGA AGTGCTATCC TAGCTGCCAC TCCTGCAGAC CCGACCCAGC CGGAATTC 1798
(2) ANGABEN ZU SEQ ID NO: 2:
   (i) SEQUENZKENNZEICHEN:
      (A) LÄNGE: 480 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 2:
   Leu Ile Pro Ala Glu Glu Glu Asn Pro Ala Phe Trp Asn Arg Gln Ala
   1               5                   10                  15
   Ala Gln Ala Leu Asp Val Ala Lys Lys Leu Gln Pro Ile Gln Thr Ala
   20 25 30
   Ala Lys Asn Val Ile Leu Phe Leu Gly Asp Gly Met Gly Val Pro Thr
   35 40 45
   Val Thr Ala Thr Arg Ile Leu Lys Gly Gln Met Asn Gly Lys Leu Gly
   50 55 60
   Pro Glu Thr Pro Leu Ala Met Asp Gln Phe Pro Tyr Val Ala Leu Ser
   65 70 75 80
   Lys Thr Tyr Asn Val Asp Arg Gln Val Pro Asp Ser Ala Gly Thr Ala
   85 90 95
   Thr Ala Tyr Leu Cys Gly Val Lys Gly Asn Tyr Arg Thr Ile Gly Val
   100 105 110
   Ser Ala Ala Ala Arg Tyr Asn Gln Cys Asn Thr Thr Arg Gly Asn Glu
   115 120 125
   Val Thr Ser Val Ile Asn Arg Ala Lys Lys Ala Gly Lys Ala Val Gly
   130 135 140
   Val Val Thr Thr Thr Arg Val Gln His Ala Ser Pro Ala Gly Ala Tyr
   145 150 155 160
   Ala His Thr Val Asn Arg Asn Trp Tyr Ser Asp Ala Asp Leu Pro Ala
   165 170 175
   Asp Ala Gln Lys Asn Gly Cys Gln Asp Ile Ala Ala Gln Leu Val Tyr
   180 185 190
   Asn Met Asp Ile Asp Val Ile Leu Gly Gly Gly Arg Met Tyr Met Phe
   195 200 205
   Pro Glu Gly Thr Pro Asp Pro Glu Tyr Pro Asp Asp Ala Ser Val Asn
   210 215 220
   Gly Val Arg Lys Asp Lys Gln Asn Leu Val Gln Glu Trp Gln Ala Lys
   225 230 235 240
   His Gln Gly Ala Gln Tyr Val Trp Asn Arg Thr Ala Leu Leu Gln Ala
   245 250 255
   Ala Asp Asp Ser Ser Val Thr His Leu Met Gly Leu Phe Glu Pro Ala
               260                 265                 270
   Asp Met Lys Tyr Asn Val Gln Gln Asp His Thr Lys Asp Pro Thr Leu
           275                 280                 285
   Ala Glu Met Thr Glu Ala Ala Leu Gln Val Leu Ser Arg Asn Pro Arg
       290                 295                 300
   Gly Phe Tyr Leu Phe Val Glu Gly Gly Arg Ile Asp His Gly His His
   305                 310                 315                 320
   Asp Gly Lys Ala Tyr Met Ala Leu Thr Glu Ala Ile Met Phe Asp Asn
                   325                 330                 335
   Ala Ile Ala Lys Ala Asn Glu Leu Thr Ser Glu Leu Asp Thr Leu Ile
               340                 345                 350
   Leu Val Thr Ala Asp His Ser His Val Phe Ser Phe Gly Gly Tyr Thr
           355                 360                 365
   Leu Arg Gly Thr Ser Ile Phe Gly Leu Ala Pro Gly Lys Ala Leu Asp
       370                 375                 380
   Ser Lys Ser Tyr Thr Ser Ile Leu Tyr Gly Asn Gly Pro Gly Tyr Ala
   385                 390                 395                 400
   Leu Gly Gly Gly Ser Arg Pro Asp Val Asn Gly Ser Thr Ser Glu Glu
                   405                 410                 415
   Pro Ser Tyr Arg Gln Gln Ala Ala Val Pro Leu Ala Ser Glu Thr His
               420                 425                 430
   Gly Gly Glu Asp Val Ala Val Phe Ala Arg Gly Pro Gln Ala His Leu
           435                 440                 445
   Val His Gly Val Gln Glu Glu Thr Phe Val Ala His Ile Met Ala Phe
       450                 455                 460
   Ala Gly Cys Val Glu Pro Tyr Thr Asp Cys Asn Leu Pro Ala Pro Ala
   465                 470                 475                 480
(2) ANGABEN ZU SEQ ID NO: 3:
   (i) SEQUENZKENNZEICHEN:
      (A) LÄNGE: 2460 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
   GAATTCGGCA CGAGCGAGAC CCAGACTCCC CAGGTCCCAT CCTGACCCTC CGCCATCACA    60
   CAGCTATGCA GGGGGCCTGC GTGCTGCTGC TGCTGGGCCT GTGGCTACAG CTCTCCCTCG 120
   CCTTCATCCC AGTTGAGGAG GAAGACCCCG CCTTCTGGAA CCGCCAGGCA GCCCAGGCCC 180
   TTGATGTGGC TAAGAAGCTG CAGCCCATCC AGAAAGCCGC CAAGAATGTC ATCCTCTTCT 240
   TGGGAGATGG GATGGGGGTG CCTACGGTGA CAGCCACTCG GATACTGAAG GGGCAGATGA 300
   ATGACAAGCT GGGACCTGAG ACACCCCTGG CCATGGACCA GTTCCCATAC GTGGCTCTGT 360
   CCAAGACATA CAACGTGGAC AGACAGGTGC CAGACAGCGC AGGCACTGCC ACTGCCTACC 420
   TGTGTGGGGT CAAGGGCAAC TACAGAACCA TCGGTGTAAG TGCAGCCGCC CGCTACAATC 480
   AGTGCAACAC GACACGTGGG AATGAGGTCA CGTCTGTGAT GAACCGGGCC AAGAAAGCAG 540
   GGAAGTCAGT GGGAGTGGTG ACCACCACCA GGGTGCAGCA CGCCTCCCCA GCCGGTGCTT 600
   ATGCACACAC GGTGAACCGT GACTGGTACT CAGACGCCGA CCTGCCTGCC GATGCACAGA 660
   CGTATGGCTG CCAGGACATC GCCACACAAC TGGTCAACAA CATGGATATT GACGTGATCC 720
   TGGGTGGAGG CCGAAAGTAC ATGTTTCCTG AGGGGACCCC AGACCCTGAA TACCCACACG 780
   ATGCCAGTGT GAATGGAGTC CGGAAGGACA AGCGGAATCT GGTGCAGGAG TGGCAGGCCA 840
   AGCACCAGGG AGCCCAGTAT GTGTGGAACC GCACGGAGCT CCTTCAGGCA GCCAATGACT 900
   CCAGTGTTAC ACATCTCATG GGCCTCTTTG AGCCGGCAGA CATGAAGTAT AATGTTCAGC 960
   AAGACCCCAC CAAGGACCCG ACCCTGGAGG AGATGACGGA GGCGGCCCTG CAAGTGCTGA 1020
   GCAGGAACCC CCAGGGCTTC TACCTCTTCG TGGAGGGAGG CCGCATTGAC CACGGTCACC 1080
   ATGATAGCAA AGCTTATATG GCGCTGACTG AGGCGGTCAT GTTTGACAAT GCCATCGCCA 1140
   AGGCTAACGA GCTCACTAGC GAACTGGACA CGCTGATCCT TGTCACTGCA GACCACTCCC 1200
   ATGTCTTCTC TTTTGGTGGC TACACACTGC GTGGGACCTC CATTTTCGGT CTGGCCCCCA 1260
   GCAAGGCCTC AGACAAGAAG TCCTACACCT CCATCCTCTA TGGCAATGGC CCTGGCTACG 1320
   TGCTTGGTGG GGGCTCAAGG CCCGATGTTA ATGACAGCAT AAGCGAGGAC CCCTCATACC 1380
   GGCAGCAGGC GGCCGTGCCC CTGTCTAGCG AGACCCACGG GGGCGAAGAC GTGGCGGTGT 1440
   TCGCGCGAGG CCCGCAGGCG CACCTGGTGC ACGGCGTGCA GGAGGAGACC TTCGTGGCGC 1500
   ACGTCATGGC CTTTGCGGGC TGCGTGGAGC CCTACACCGA CTGCAATCTG CCGGCCCCCT 1560
   CTGGCCTCTC CGACGCCGCG CACCTGGCGG CCAGCGCGCC TTCGCTAGCG CTGCTGGCCG 1620
   GGGCGATGCT GCTGCTGCTG GCGCCCGCCT TGTACTGACC CCCACCAACT CCAGGTCTTG 1680
   GGGTTTCCCG CTTTCTTGCC CCAAAATCTC CCAGCGCAGG CCCCATCTGA GCTACCACCT 1740
   CAGAGTCCCC ACCCTGAAGT CCTATCTAGC GCACTCCAGA CCGCGACTCA GCCCCACCAC 1800
   CAGAGCTTCA CCTCCCAGCA ACGAAGGAGC CTTAGCTCAC AGCCTTTCAT GGCCCAGACC 1860
   ATTCTGGAGA CTGAGGCCCT GATTTTCCCG ACCCAACTTC AGTGGCTTGA GATTTTGTGT 1920
   TCTGCCACCC CGGATCCCTG TAAGGGGGCT CGGACCATCC AGACTCCCCC CACTGCCCAC 1980
   AGCCGAACCT GAGGACCAGG CTGGCACGGT CCCAGGGGTC CCAGGCCCGG CTGGAACCCA 2040
   CATCTTTGCC TTTCAGGAGA CCCTGGGACT GTGGGGTTTC CAGGAGGCGT GGCTTCTTGG 2100
   AGGCGTGGCT TCGGAGGGGT GGCTTCCGAG AAGGCGTGGC TCCCTGTCCT GGAACCACCC 2160
   TGTGGGNATC TGGGGCCCAA GGAGATGTCT GGGGCAAAGA GTGCCGGGGG ACCCTGGACA 2220
   CAGAATCTTC AGCGGCCCCT CCTAGGAACC CAGCAGTACC ATTATAGAGA GGGGACACCG 2280
   ACACAGAGGA GAGGAGACTT GTCCCAGGTC CCTCAGCTGC TGTGAGGGGT GACCCTTGGT 2340
   TCCCGTTACC AGGCTGGGGG ATCCCAGGAG CAGCGGGGGA CCTGGGGGTG GGGACACAGG 2400
   CCCCACACTC CTGGGAGGGA GGAAGCAGCC CTNAAATAAA CTGTTCCTCG TGCCGAATTC 2460
(2) ANGABEN ZU SEQ ID NO: 4:
   (i) SEQUENZKENNZEICHEN:
      (A) LANGE: 511 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 4:
   Phe Ile Pro Val Glu Glu Glu Asp Pro Ala Phe Trp Asn Arg Gln Ala
   1               5                   10                  15
   Ala Gln Ala Leu Asp Val Ala Lys Lys Leu Gln Pro Ile Gln Lys Ala
               20                  25                  30
   Ala Lys Asn Val Ile Leu Phe Leu Gly Asp Gly Met Gly Val Pro Thr
           35                  40                  45
   Val Thr Ala Thr Arg Ile Leu Lys Gly Gln Met Asn Asp Lys Leu Gly
   50 55 60
   Pro Glu Thr Pro Leu Ala Met Asp Gln Phe Pro Tyr Val Ala Leu Ser
   65 70 75 80
   Lys Thr Tyr Asn Val Asp Arg Gln Val Pro Asp Ser Ala Gly Thr Ala
   85 90 95
   Thr Ala Tyr Leu Cys Gly Val Lys Gly Asn Tyr Arg Thr Ile Gly Val
   100 105 110
   Ser Ala Ala Ala Arg Tyr Asn Gln Cys Asn Thr Thr Arg Gly Asn Glu
   115 120 125
   Val Thr Ser Val Met Asn Arg Ala Lys Lys Ala Gly Lys Ser Val Gly
   130 135 140
   Val Val Thr Thr Thr Arg Val Gln His Ala Ser Pro Ala Gly Ala Tyr
   145 150 155 160
   Ala His Thr Val Asn Arg Asp Trp Tyr Ser Asp Ala Asp Leu Pro Ala
   165 170 175
   Asp Ala Gln Thr Tyr Gly Cys Gln Asp Ile Ala Thr Gln Leu Val Asn
   180 185 190
   Asn Met Asp Ile Asp Val Ile Leu Gly Gly Gly Arg Lys Tyr Met Phe
   195 200 205
   Pro Glu Gly Thr Pro Asp Pro Glu Tyr Pro His Asp Ala Ser Val Asn
   210 215 220
   Gly Val Arg Lys Asp Lys Arg Asn Leu Val Gln Glu Trp Gln Ala Lys
   225 230 235 240
   His Gln Gly Ala Gln Tyr Val Trp Asn Arg Thr Glu Leu Leu Gln Ala
   245 250 255
   Ala Asn Asp Ser Ser Val Thr His Leu Met Gly Leu Phe Glu Pro Ala
   260 265 270
   Asp Met Lys Tyr Asn Val Gln Gln Asp Pro Thr Lys Asp Pro Thr Leu
   275 280 285
   Glu Glu Met Thr Glu Ala Ala Leu Gln Val Leu Ser Arg Asn Pro Gln
   290 295 300
   Gly Phe Tyr Leu Phe Val Glu Gly Gly Arg Ile Asp His Gly His His
   305 310 315 320
   Asp Ser Lys Ala Tyr Met Ala Leu Thr Glu Ala Val Met Phe Asp Asn
   325 330 335
   Ala Ile Ala Lys Ala Asn Glu Leu Thr Ser Glu Leu Asp Thr Leu Ile
               340                 345                 350
   Leu Val Thr Ala Asp His Ser His Val Phe Ser Phe Gly Gly Tyr Thr
           355                 360                 365
   Leu Arg Gly Thr Ser Ile Phe Gly Leu Ala Pro Ser Lys Ala Ser Asp
       370                 375                 380
   Lys Lys Ser Tyr Thr Ser Ile Leu Tyr Gly Asn Gly Pro Gly Tyr Val
   385                 390                 395                 400
   Leu Gly Gly Gly Ser Arg Pro Asp Val Asn Asp Ser Ile Ser Glu Asp
                   405                 410                 415
   Pro Ser Tyr Arg Gln Gln Ala Ala Val Pro Leu Ser Ser Glu Thr His
               420                 425                 430
   Gly Gly Glu Asp Val Ala Val Phe Ala Arg Gly Pro Gln Ala His Leu
           435                 440                 445
   Val His Gly Val Gln Glu Glu Thr Phe Val Ala His Val Met Ala Phe
       450                 455                 460
   Ala Gly Cys Val Glu Pro Tyr Thr Asp Cys Asn Leu Pro Ala Pro Ser
   465                 470                 475                 480
   Gly Leu Ser Asp Ala Ala His Leu Ala Ala Ser Ala Pro Ser Leu Ala
                   485                 490                 495
   Leu Leu Ala Gly Ala Met Leu Leu Leu Leu Ala Pro Ala Leu Tyr
               500                 505                 510
(2) ANGABEN ZU SEQ ID NO: 5:
   (i) SEQUENZKENNZEICHEN:
      (A) LÄNGE: 2542 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
   GAATTCGGCA CGAGGAGACC CGGCCTCCCC AGGTCCCATC CTGACCCTCC GCCATCACAC    60
   AGCCATGCAG TGGGCCTGTG TGCTGCTGCT GCTGGGCCTG TGGCTACAGC TCTCCCTCAC   120
   CTTCATCCCA GCTGAGGAGG AAGACCCCGC CTTCTGGAAC CGCCAGGCAG CCCAGGCCCT   180
   TGATGTAGCC AAGAAGTTGC AGCCGATCCA GACAGCTGCC AAGAATGTCA TCCTCTTCTT   240
   GGGGGATGGG ATGGGGGTGC CTACGGTGAC AGCCACTCGG ATCCTAAAGG GGCAGATGAA  300
   TGGTAAGCTG GGACCTGAGA CACCCCTGGC CATGGACCAG TTCCCATACG TGGCTCTGTC  360
   CAAGACATAC AACGTGGACA GACAGGTGCC AGACAGCGCA GGCACTGCCA CTGCCTACCT  420
   GTGTGGGGTC AAGGGCAACT ACAAAACCAT TGGTGTAAGT GCAGCCGCCC GCTACAACCA  480
   GTGCAACACA ACAAGTGGCA ATGAGGTCAC GTCTGTGATG AACCGGGCCA AGAAAGCAGG  540
   AAAGTCAGTG GGAGTGGTGA CCACCTCCAG GGTGCAGCAT GCCTCCCCAG CCGGTGCTTA  600
   TGCACACACG GTGAACCGAA ACTGGTACTC AGATGCCGAC CTGCCTGCCG ATGCACAGAC  660
   GTATGGCTGC CAGGACATCG CCACACAACT GGTCAACAAC ATGGATATTG ACGTGATCCT  720
   GGGTGGAGGC CGAATGTACA TGTTTCCTGA GGGGACCCCG GATCCTGAAT ACCCATACGA  780
   TGTCAATCAG ACTGGAGTCC GGAAGGACAA GCGGAATCTG GTGCAGGAGT GGCAGGCCAA  840
   GCACCAGGGA GCCCAGTATG TGTGGAACCG CACGGAGCTC CTTCAGGCAG CCAATGACCC  900
   CAGTGTAACA CACCTCATGG GCCTCTTTGA GCCGGCAGAC ATGAAGTATA ATGTTCAGCA  960
   AGACCCCACC AAGGACCCGA CCCTGGAGGA GATGACGGAG GCGGCCCTGC AAGTGCTGAG 1020
   CAGGAACCCC CAGGGCTTCT ACCTCTTCGT GGAGGGAGGC CGCATTGACC ACGGTCACCA 1080
   TGAAGGCAAA GCTTATATGG CACTGACTGA TACAGTCATG TTTGACAATG CCATCGCCAA 1140
   GGCTAACGAG CTCACTAGCG AACTGGACAC GCTGATCCTT GCCACTGCAG ACCACTCCCA 1200
   TGTCTTCTCT TTTGGTGGCT ACACACTGCG TGGGACCTCC ATTTTCGGTC TGGCCCCCAG 1260
   CAAGGCCTCA GACAACAAGT CCTACACCTC CATCCTCTAT GGCAATGGCC CTGGCTACGT 1320
   GCTTGGTGGG GGCTTAAGGC CCGATGTTAA TGACAGCATA AGCGAGGACC CCTCGTACCG 1380
   GCAGCAGGCG GCCGTGCCCC TGTCTAGTGA GTCCCACGGG GGCGAGGACG TGGCGGTGTT 1440
   CGCGCGAGGC CCGCAGGCGC ACCTGGTGCA CGGCGTGCAG GAGGAGACCT TCGTGGCGCA 1500
   CGTCATGGCC TTTGCGGGCT GCGTGGAGCC CTACACCGAC TGCAATCTGC CGGCCCCCTC 1560
   TGGCCTCTCC GACGCCGCGC ACCTGGCGGC CAGCCCGCCT TCGCTGGCGC TGCTGGCCGG 1620
   GGCGATGCTG CTGCTGCTGG CGCCTGCCTT GTACTGACCC CCACCAACTC CAGGTCTTGG 1680
   GGTTTCCTGC TTTCCTGCCA AAAATCTCCC AGCGCAGACC CCACCAGAGC TACCACCTCG 1740
   GAGTCTCCAC CCTGAAGTCC TATCTTAGCG GCCACTCCCG GATCCCCGAC CAGGCCCCAC 1800
   TAGCAGAGCT TCACCTCCCA GAAATGAAGG ATTCACCTTC CAGCAACGAA GAAGCCTCAG 1860
   CTCACAGCCC TTCATGGCCC AGCCCATCCA GAGGCTGAGG CCCTGATTTC CCTGTGACAC 1920
   CCGTAGACCT ACTGCCCGAC CCCAACTTCA GTGGCTTGGG ATTTTGTGTT CTGCCACCCC 1980
   TAACCCCAGT AAGGGGGCTC GGACCATCCA GACTCTCCCC ACTGCCCACA ACCCCACCTG 2040
   AGAACCAGGC TAGCACGGTC CCAAGGTTCC CAGGCCCGGC TAGAACCCAC ACCATGCCTT 2100
   TCAGGAGACC CTGGGGCTCC GGGGTTTCCG GGAGGCGTGG CTTTCTTAGG AGGCGTGGAA 2160
   ACTGAGGAGG CACGGTTTCT GAGGAGGCGT GCGTCCTGGG GAGCTGTGGC TTCCGGTCCT 2220
   CCCCATGCCC TGTGGGCTCC TCCCTAACCA AGGAGACGGC CAAGGAGACG TCTGGAACCA 2280
   GGAGCGGCGG GGGAACCTTG CAGAGCCCTC AGCAACCCCT CCTAGGAACC CAGGGTACCG 2340
   TTAGAGAGAG GAGACAGCGA CACAGAGGAG AGGAGACTTG TCCCAGGTCT CTCAGCTGCT 2400
   ATGAAGGTGG CCCCGGTGCC CCTTCCAGGC TGGGAGATCC CAGGAGCAGC GGGGGAGCTG 2460
   GTGGGTGGGG ACACAGCCCC GCCTTCATGG GAGGGAGGAA GCAGCCCTCA AATAAACTGT 2520
   TCTAAGTGTG AAAAAATCTA GA                                         2542
(2) ANGABEN ZU SEQ ID NO: 6:
   (i) SEQUENZKENNZEICHEN:
      (A) LÄNGE: 511 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 6:
   Phe Ile Pro Ala Glu Glu Glu Asp Pro Ala Phe Trp Asn Arg Gln Ala
   1               5                   10                  15
   Ala Gln Ala Leu Asp Val Ala Lys Lys Leu Gln Pro Ile Gln Thr Ala
               20                  25                  30
   Ala Lys Asn Val Ile Leu Phe Leu Gly Asp Gly Met Gly Val Pro Thr
           35                  40                  45
   Val Thr Ala Thr Arg Ile Leu Lys Gly Gln Met Asn Gly Lys Leu Gly
       50                  55                  60
   Pro Glu Thr Pro Leu Ala Met Asp Gln Phe Pro Tyr Val Ala Leu Ser
   65                  70                  75                  80
   Lys Thr Tyr Asn Val Asp Arg Gln Val Pro Asp Ser Ala Gly Thr Ala
   85 90 95
   Thr Ala Tyr Leu Cys Gly Val Lys Gly Asn Tyr Lys Thr Ile Gly Val
   100 105 110
   Ser Ala Ala Ala Arg Tyr Asn Gln Cys Asn Thr Thr Ser Gly Asn Glu
   115 120 125
   Val Thr Ser Val Met Asn Arg Ala Lys Lys Ala Gly Lys Ser Val Gly
   130 135 140
   Val Val Thr Thr Ser Arg Val Gln His Ala Ser Pro Ala Gly Ala Tyr
   145 150 155 160
   Ala His Thr Val Asn Arg Asn Trp Tyr Ser Asp Ala Asp Leu Pro Ala
   165 170 175
   Asp Ala Gln Thr Tyr Gly Cys Gln Asp Ile Ala Thr Gln Leu Val Asn
   180 185 190
   Asn Met Asp Ile Asp Val Ile Leu Gly Gly Gly Arg Met Tyr Met Phe
   195 200 205
   Pro Glu Gly Thr Pro Asp Pro Glu Tyr Pro Tyr Asp Val Asn Gln Thr
   210 215 220
   Gly Val Arg Lys Asp Lys Arg Asn Leu Val Gln Glu Trp Gln Ala Lys
   225 230 235 240
   His Gln Gly Ala Gln Tyr Val Trp Asn Arg Thr Glu Leu Leu Gln Ala
   245 250 255
   Ala Asn Asp Pro Ser Val Thr His Leu Met Gly Leu Phe Glu Pro Ala
   260 265 270
   Asp Met Lys Tyr Asn Val Gln Gln Asp Pro Thr Lys Asp Pro Thr Leu
   275 280 285
   Glu Glu Met Thr Glu Ala Ala Leu Gln Val Leu Ser Arg Asn Pro Gln
   290 295 300
   Gly Phe Tyr Leu Phe Val Glu Gly Gly Arg Ile Asp His Gly His His
   305 310 315 320
   Glu Gly Lys Ala Tyr Met Ala Leu Thr Asp Thr Val Met Phe Asp Asn
   325 330 335
   Ala Ile Ala Lys Ala Asn Glu Leu Thr Ser Glu Leu Asp Thr Leu Ile
   340 345 350
   Leu Ala Thr Ala Asp His Ser His Val Phe Ser Phe Gly Gly Tyr Thr
   355 360 365
   Leu Arg Gly Thr Ser Ile Phe Gly Leu Ala Pro Ser Lys Ala Ser Asp
       370                 375                 380
   Asn Lys Ser Tyr Thr Ser Ile Leu Tyr Gly Asn Gly Pro Gly Tyr Val
   385                 390                 395                     400
   Leu Gly Gly Gly Leu Arg Pro Asp Val Asn Asp Ser Ile Ser Glu Asp
                   405                 410                     415
   Pro Ser Tyr Arg Gln Gln Ala Ala Val Pro Leu Ser Ser Glu Ser His
               420                 425                 430
   Gly Gly Glu Asp Val Ala Val Phe Ala Arg Gly Pro Gln Ala His Leu
           435                 440                 445
   Val His Gly Val Gln Glu Glu Thr Phe Val Ala His Val Met Ala Phe
       450                 455                 460
   Ala Gly Cys Val Glu Pro Tyr Thr Asp Cys Asn Leu Pro Ala Pro Ser
   465                 470                 475                     480
   Gly Leu Ser Asp Ala Ala His Leu Ala Ala Ser Pro Pro Ser Leu Ala
                   485                 490                     495
   Leu Leu Ala Gly Ala Met Leu Leu Leu Leu Ala Pro Ala Leu Tyr
               500                 505                 510
(2) ANGABEN ZU SEQ ID NO: 7:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) BESCHREIBUNG: /desc = "Oligonucleotid"
(2) ANGABEN ZU SEQ ID NO: 8:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 8:
   GAGGATGACA TTCTTGGC 18
(2) ANGABEN ZU SEQ ID NO: 9:
   (i) SEQUENZKENNZEICHEN:
      (A) LÄNGE: 17 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 9:
   GGTGTAAGTG CAGCCGC 17
(2) ANGABEN ZU SEQ ID NO: 10:
   (i) SEQUENZKENNZEICHEN:
      (A) LANGE: 17 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 10:
   GCGGCTGCAC TTAGACC 17
(2) ANGABEN ZU SEQ ID NO: 11:
   (i) SEQUENZKENNZEICHEN:
      (A) LÄNGE: 17 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 11:
   AATGTACATG TTTCCTG 17
(2) ANGABEN ZU SEQ ID NO: 12:
   (i) SEQUENZKENNZEICHEN:
      (A) LÄNGE: 17 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 12:
   CAGGAAACAT GTACATT 17
(2) ANGABEN ZU SEQ ID NO: 13:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 13:
   CCAGGGCTTC TACCTCTT 18
(2) ANGABEN ZU SEQ ID NO: 14:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 14:
   AAGAGGTAGA AGCCCTGG 18
(2) ANGABEN ZU SEQ ID NO: 15:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 15:
   ACCAGAGCTA CCACCTCG 18
(2) ANGABEN ZU SEQ ID NO: 16:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 16:
   AAGCAGGAAA CCCCAAGA 18
(2) ANGABEN ZU SEQ ID NO: 17:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 17:
   CTTCAGTGGC TTGGGATT 18
(2) ANGABEN ZU SEQ ID NO: 18:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 18:
   AATCCCAAGC CACTGAAG 18
(2) ANGABEN ZU SEQ ID NO: 19:
   (i) SEQUENZKENNZEICHEN:
      (A) LANGE: 17 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 19:
   CGAGGTCGAC GGTATCG 17
(2) ANGABEN ZU SEQ ID NO: 20:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 20:
   GCAGGTCTCT CAGCTGGGAT GAGGGTGAGG 30
(2) ANGABEN ZU SEQ ID NO: 21:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 21:
   GCAGGTCTCA GCTGAGGAGG AAAACCCCGC 30
(2) ANGABEN ZU SEQ ID NO: 22:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 22:
   GCAGGTCTCT GTTGTGTCGC ACTGGTT 27
(2) ANGABEN ZU SEQ ID NO: 23:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 23:
   GGTCTCTTTC TTGGCCCGGT TGATCAC 27
(2) ANGABEN ZU SEQ ID NO: 24:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 24:
   GGTCTCAAGA AAGCAGGGAA GGCCGTC 27
(2) ANGABEN ZU SEQ ID NO: 25:
   (i) SEQUENZKENNZEICHEN:
      (A) LÄNGE: 28 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 25:
   GGTCTCGTGC ATCAGCAGGC AGGTCGGC 28
(2) ANGABEN ZU SEQ ID NO: 26:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 26:
   GGTCTCATGC ACAGAAGAAT GGCTGCCAG 29
(2) ANGABEN ZU SEQ ID NO: 27:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 27:
   GGTCTCAAAC ATGTACATTC GGCCTCCACC 30
(2) ANGABEN ZU SEQ ID NO: 28:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 28:
   GTCTCCATGT TTCCTGAGGG GACCCCA 27
(2) ANGABEN ZU SEQ ID NO: 29:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 29:
   GGTCTCCTGC CATTCCTGCA CCAGGTT 27
(2) ANGABEN ZU SEQ ID NO: 30:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 30:
   GGTCTCTGGC AGGCCAAGCA CCAGGGA 27
(2) ANGABEN ZU SEQ 10 NO: 31:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 31:
(2) ANGABEN ZU SEQ ID NO: 32:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 32:
   GGTCTCGACC CTGGCGGAGA TGACG 25
(2) ANGABEN ZU SEQ ID NO: 33:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 33:
   GGTCTCCTCA GTCAGTGCCA TATA 24
(2) ANGABEN ZU SEQ ID NO: 34:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 34:
   GGTCTCACTG AGGCGATCAT GTTTGAC 27
(2) ANGABEN ZU SEQ ID NO: 35:
   (i) SEQUENZKENNZEICHEN:
      (A) LÄNGE: 23 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 35:
   TGCACCAGGT GCGCCTGCGG GCC 23
(2) ANGABEN ZU SEQ ID NO: 36:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 36:
   GCCGCACAGC TGGTCTACAA CATGGAT 27
(2) ANGABEN ZU SEQ ID NO: 37:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 37:
   GCTGTCTAAG GCCTTGCCGG GGGC 24
(2) ANGABEN ZU SEQ ID NO: 38:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 38:
   GCCGCACAGC TGGTCTACAA CATGGAT 27
(2) ANGABEN ZU SEQ ID NO: 39:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 39:
   GGGGGTCTCG CTTGCTGCCA TTAAC 25
(2) ANGABEN ZU SEQ ID NO: 40:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 40:
   GTTAATGGTC TCACAAGCGA GGAACCCTCG 30
(2) ANGABEN ZU SEQ ID NO: 41:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 41:
   CCCGTGGGTC TCGCTAGCCA GGGGCAC 27
(2) ANGABEN ZU SEQ ID NO: 42:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 42:
   GTTAATGGTC TCACAAGCGA GGAACCCTCG 30
(2) ANGABEN ZU SEQ ID NO: 43:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 43:
   GATGCTGGTC TCGGTGGAGG GGGCTGGCAG 30
(2) ANGABEN ZU SEQ ID NO: 44:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 44:
   CTGCCAGGTC TCACCACCGC CACCAGCATC 30
(2) ANGABEN ZU SEQ ID NO: 45:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 45:
   CATACGATTT AGGTGACACT ATAG 24
(2) ANGABEN ZU SEQ ID NO: 46:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 46:
   GGTCTCTGGC AGGCCAAGCA CCAGGGA 27
(2) ANGABEN ZU SEQ ID NO: 47:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 47:
   GTAGAAGCCC CGGGGGTTCC TGCT 24
(2) ANGABEN ZU SEQ ID NO: 48:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 48:
   AGCAGGAACC CCCGGGGCTT CTAC 24
(2) ANGABEN ZU SEQ ID NO: 49:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 49:
   TGCCATATAA GCTTTGCCGT CATGGTG 27
(2) ANGABEN ZU SEQ ID NO: 50:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 50:
   GGTCTCTTTC TTGGCCCGGT TCATCAC 27
(2) ANGABEN ZU SEQ ID NO: 51:
   (i) SEQUENZKENNZEICHEN:
      (A) LANGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 51:
   TGGTCACCAC TCCCACGGAC TTCCCTG 27
(2) ANGABEN ZU SEQ ID NO: 52:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 52:
   GGTCTCAAAC ATGTATTTTC GGCCTCCACC 30
(2) ANGABEN ZU SEQ ID NO: 53:
   (i) SEQUENZKENNZEICHEN:
      (A) LANGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 53:
   GGTCTCATGT TTCCTGTGGG GACCCCAGAC 30
(2) ANGABEN ZU SEQ ID NO: 54:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonukleotid"
   (xi) SEQUENZBESCHREIBUNG: SEQ ID NO: 54:
   GGTCTCCTGC CATGCCTGCA CCAGGTT 27

### SEQUENCE LISTING

<110> Roche Diagnostics GmbH
<120> Hochaktive alkalische Phosphatase
<130> 489300ep
<140> 99108502.8-2105
   <141> 1999-04-30
<160> 54
<170> PatentIn Ver. 2.1
<210> 1
   <211> 1798
   <212> DNA
   <213> Bovine intestinal
<400> 1
<210> 2
   <211> 480
   <212> PRT
   <213> Bovine intestinal
<400> 2
   Leu Ile Pro Ala Glu Glu Glu Asn Pro Ala Phe Trp Asn Arg Gln Ala
    1               5                  10                  15
   Ala Gln Ala Leu Asp Val Ala Lys Lys Leu Gln Pro Ile Gln Thr Ala
               20                  25                  30
   Ala Lys Asn Val Ile Leu Phe Leu Gly Asp Gly Met Gly Val Pro Thr
           35                  40                  45
   Val Thr Ala Thr Arg Ile Leu Lys Gly Gln Met Asn Gly Lys Leu Gly
       50                  55                  60
   Pro Glu Thr Pro Leu Ala Met Asp Gln Phe Pro Tyr Val Ala Leu Ser
   65                  70                  75                  80
   Lys Thr Tyr Asn Val Asp Arg Gln Val Pro Asp Ser Ala Gly Thr Ala
                   85                  90                  95
   Thr Ala Tyr Leu Cys Gly Val Lys Gly Asn Tyr Arg Thr Ile Gly Val
               100                 105                 110
   Ser Ala Ala Ala Arg Tyr Asn Gln Cys Asn Thr Thr Arg Gly Asn Glu
           115                 120                 125
   Val Thr Ser Val Ile Asn Arg Ala Lys Lys Ala Gly Lys Ala Val Gly
       130                 135                 140
   Val Val Thr Thr Thr Arg Val Gln His Ala Ser Pro Ala Gly Ala Tyr
   145                 150                 155                 160
   Ala His Thr Val Asn Arg Asn Trp Tyr Ser Asp Ala Asp Leu Pro Ala
                   165                 170                 175
   Asp Ala Gln Lys Asn Gly Cys Gln Asp Ile Ala Ala Gln Leu Val Tyr
               180                 185                 190
   Asn Met Asp Ile Asp Val Ile Leu Gly Gly Gly Arg Met Tyr Met Phe
           195                 200                 205
   Pro Glu Gly Thr Pro Asp Pro Glu Tyr Pro Asp Asp Ala Ser Val Asn
   210 215 220
   Gly Val Arg Lys Asp Lys Gln Asn Leu Val Gln Glu Trp Gln Ala Lys
   225 230 235 240
   His Gln Gly Ala Gln Tyr Val Trp Asn Arg Thr Ala Leu Leu Gln Ala
   245 250 255
   Ala Asp Asp Ser Ser Val Thr His Leu Met Gly Leu Phe Glu Pro Ala
   260 265 270
   Asp Met Lys Tyr Asn Val Gln Gln Asp His Thr Lys Asp Pro Thr Leu
   275 280 285
   Ala Glu Met Thr Glu Ala Ala Leu Gln Val Leu Ser Arg Asn Pro Arg
   290 295 300
   Gly Phe Tyr Leu Phe Val Glu Gly Gly Arg Ile Asp His Gly His His
   305 310 315 320
   Asp Gly Lys Ala Tyr Met Ala Leu Thr Glu Ala Ile Met Phe Asp Asn
   325 330 335
   Ala Ile Ala Lys Ala Asn Glu Leu Thr Ser Glu Leu Asp Thr Leu Ile
   340 345 350
   Leu Val Thr Ala Asp His Ser His Val Phe Ser Phe Gly Gly Tyr Thr
   355 360 365
   Leu Arg Gly Thr Ser Ile Phe Gly Leu Ala Pro Gly Lys Ala Leu Asp
   370 375 380
   Ser Lys Ser Tyr Thr Ser Ile Leu Tyr Gly Asn Gly Pro Gly Tyr Ala
   385 390 395 400
   Leu Gly Gly Gly Ser Arg Pro Asp Val Asn Gly Ser Thr Ser Glu Glu
   405 410 415
   Pro Ser Tyr Arg Gln Gln Ala Ala Val Pro Leu Ala Ser Glu Thr His
   420 425 430
   Gly Gly Glu Asp Val Ala Val Phe Ala Arg Gly Pro Gln Ala His Leu
   435 440 445
   Val His Gly Val Gln Glu Glu Thr Phe Val Ala His Ile Met Ala Phe
   450 455 460
   Ala Gly Cys Val Glu Pro Tyr Thr Asp Cys Asn Leu Pro Ala Pro Ala
   465 470 475 480
<210> 3
   <211> 2460
   <212> DNA
   <213> Bovine intestinal
<400> 3
   gaattcggca cgagcgagac ccagactccc caggtcccat cctgaccctc cgccatcaca 60
   cagctatgca gggggcctgc gtgctgctgc tgctgggcct gtggctacag ctctccctcg 120
   ccttcatccc agttgaggag gaagaccccg ccttctggaa ccgccaggca gcccaggccc 180
   ttgatgtggc taagaagctg cagcccatcc agaaagccgc caagaatgtc atcctcttct 240
   tgggagatgg gatgggggtg cctacggtga cagccactcg gatactgaag gggcagatga 300
   atgacaagct gggacctgag acacccctgg ccatggacca gttcccatac gtggctctgt 360
   ccaagacata caacgtggac agacaggtgc cagacagcgc aggcactgcc actgcctacc 420
   tgtgtggggt caagggcaac tacagaacca tcggtgtaag tgcagccgcc cgctacaatc 480
   agtgcaacac gacacgtggg aatgaggtca cgtctgtgat gaaccgggcc aagaaagcag 540
   ggaagtcagt gggagtggtg accaccacca gggtgcagca cgcctcccca gccggtgctt 600
   atgcacacac ggtgaaccgt gactggtact cagacgccga cctgcctgcc gatgcacaga 660
   cgtatggctg ccaggacatc gccacacaac tggtcaacaa catggatatt gacgtgatcc 720
   tgggtggagg ccgaaagtac atgtttcctg aggggacccc agaccctgaa tacccacacg 780
   atgccagtgt gaatggagtc cggaaggaca agcggaatct ggtgcaggag tggcaggcca 840
   agcaccaggg agcccagtat gtgtggaacc gcacggagct ccttcaggca gccaatgact 900
   ccagtgttac acatctcatg ggcctctttg agccggcaga catgaagtat aatgttcagc 960
   aagaccccac caaggacccg accctggagg agatgacgga ggcggccctg caagtgctga 1020
   gcaggaaccc ccagggcttc tacctcttcg tggagggagg ccgcattgac cacggtcacc 1080
   atgatagcaa agcttatatg gcgctgactg aggcggtcat gtttgacaat gccatcgcca 1140
   aggctaacga gctcactagc gaactggaca cgctgatcct tgtcactgca gaccactccc 1200
   atgtcttctc ttttggtggc tacacactgc gtgggacctc cattttcggt ctggccccca 1260
   gcaaggcctc agacaagaag tcctacacct ccatcctcta tggcaatggc cctggctacg 1320
   tgcttggtgg gggctcaagg cccgatgtta atgacagcat aagcgaggac ccctcatacc 1380
   ggcagcaggc ggccgtgccc ctgtctagcg agacccacgg gggcgaagac gtggcggtgt 1440
   tcgcgcgagg cccgcaggcg cacctggtgc acggcgtgca ggaggagacc ttcgtggcgc 1500
   acgtcatggc ctttgcgggc tgcgtggagc cctacaccga ctgcaatctg ccggccccct 1560
   ctggcctctc cgacgccgcg cacctggcgg ccagcgcgcc ttcgctagcg ctgctggccg 1620
   gggcgatgct gctgctgctg gcgcccgcct tgtactgacc cccaccaact ccaggtcttg 1680
   gggtttcccg ctttcttgcc ccaaaatctc ccagcgcagg ccccatctga gctaccacct 1740
   cagagtcccc accctgaagt cctatctagc gcactccaga ccgcgactca gccccaccac 1800
   cagagcttca cctcccagca acgaaggagc cttagctcac agcctttcat ggcccagacc 1860
   attctggaga ctgaggccct gattttcccg acccaacttc agtggcttga gattttgtgt 1920
   tctgccaccc cggatccctg taagggggct cggaccatcc agactccccc cactgcccac 1980
   agccgaacct gaggaccagg ctggcacggt cccaggggtc ccaggcccgg ctggaaccca 2040
   catctttgcc tttcaggaga ccctgggact gtggggtttc caggaggcgt ggcttcttgg 2100
   aggcgtggct tcggaggggt ggcttccgag aaggcgtggc tccctgtcct ggaaccaccc 2160
   tgtgggnatc tggggcccaa ggagatgtct ggggcaaaga gtgccggggg accctggaca 2220
   cagaatcttc agcggcccct cctaggaacc cagcagtacc attatagaga ggggacaccg 2280
   acacagagga gaggagactt gtcccaggtc cctcagctgc tgtgaggggt gacccttggt 2340
   tcccgttacc aggctggggg atcccaggag cagcggggga cctgggggtg gggacacagg 2400
   ccccacactc ctgggaggga ggaagcagcc ctnaaataaa ctgttcctcg tgccgaattc 2460
<210> 4
   <211> 511
   <212> PRT
   <213> Bovine intestinal
<400> 4
   Phe Ile Pro Val Glu Glu Glu Asp Pro Ala Phe Trp Asn Arg Gln Ala
   1               5                   10                  15
   Ala Gln Ala Leu Asp Val Ala Lys Lys Leu Gln Pro Ile Gln Lys Ala
               20                  25                  30
   Ala Lys Asn Val Ile Leu Phe Leu Gly Asp Gly Met Gly Val Pro Thr
           35                  40                  45
   Val Thr Ala Thr Arg Ile Leu Lys Gly Gln Met Asn Asp Lys Leu Gly
       50                  55                  60
   Pro Glu Thr Pro Leu Ala Met Asp Gln Phe Pro Tyr Val Ala Leu Ser
   65                  70                  75                  80
   Lys Thr Tyr Asn Val Asp Arg Gln Val Pro Asp Ser Ala Gly Thr Ala
                   85                  90                  95
   Thr Ala Tyr Leu Cys Gly Val Lys Gly Asn Tyr Arg Thr Ile Gly Val
               100                 105                 110
   Ser Ala Ala Ala Arg Tyr Asn Gln Cys Asn Thr Thr Arg Gly Asn Glu
           115                 120                 125
   Val Thr Ser Val Met Asn Arg Ala Lys Lys Ala Gly Lys Ser Val Gly
       130                 135                 140
   Val Val Thr Thr Thr Arg Val Gln His Ala Ser Pro Ala Gly Ala Tyr
   145                 150                 155                 160
   Ala His Thr Val Asn Arg Asp Trp Tyr Ser Asp Ala Asp Leu Pro Ala
                   165                 170                 175
   Asp Ala Gln Thr Tyr Gly Cys Gln Asp Ile Ala Thr Gln Leu Val Asn
   180 185 190
   Asn Met Asp Ile Asp Val Ile Leu Gly Gly Gly Arg Lys Tyr Met Phe
   195 200 205
   Pro Glu Gly Thr Pro Asp Pro Glu Tyr Pro His Asp Ala Ser Val Asn
   210 215 220
   Gly Val Arg Lys Asp Lys Arg Asn Leu Val Gln Glu Trp Gln Ala Lys
   225 230 235 240
   His Gln Gly Ala Gln Tyr Val Trp Asn Arg Thr Glu Leu Leu Gln Ala
   245 250 255
   Ala Asn Asp Ser Ser Val Thr His Leu Met Gly Leu Phe Glu Pro Ala
   260 265 270
   Asp Met Lys Tyr Asn Val Gln Gln Asp Pro Thr Lys Asp Pro Thr Leu
   275 280 285
   Glu Glu Met Thr Glu Ala Ala Leu Gln Val Leu Ser Arg Asn Pro Gln
   290 295 300
   Gly Phe Tyr Leu Phe Val Glu Gly Gly Arg Ile Asp His Gly His His
   305 310 315 320
   Asp Ser Lys Ala Tyr Met Ala Leu Thr Glu Ala Val Met Phe Asp Asn
   325 330 335
   Ala Ile Ala Lys Ala Asn Glu Leu Thr Ser Glu Leu Asp Thr Leu Ile
   340 345 350
   Leu Val Thr Ala Asp His Ser His Val Phe Ser Phe Gly Gly Tyr Thr
   355 360 365
   Leu Arg Gly Thr Ser Ile Phe Gly Leu Ala Pro Ser Lys Ala Ser Asp
   370 375 380
   Lys Lys Ser Tyr Thr Ser Ile Leu Tyr Gly Asn Gly Pro Gly Tyr Val
   385 390 395 400
   Leu Gly Gly Gly Ser Arg Pro Asp Val Asn Asp Ser Ile Ser Glu Asp
   405 410 415
   Pro Ser Tyr Arg Gln Gln Ala Ala Val Pro Leu Ser Ser Glu Thr His
   420 425 430
   Gly Gly Glu Asp Val Ala Val Phe Ala Arg Gly Pro Gln Ala His Leu
   435 440 445
   Val His Gly Val Gln Glu Glu Thr Phe Val Ala His Val Met Ala Phe
   450 455 460
   Ala Gly Cys Val Glu Pro Tyr Thr Asp Cys Asn Leu Pro Ala Pro Ser
   465 470 475 480
   Gly Leu Ser Asp Ala Ala His Leu Ala Ala Ser Ala Pro Ser Leu Ala
   485 490 495
   Leu Leu Ala Gly Ala Met Leu Leu Leu Leu Ala Pro Ala Leu Tyr
   500 505 510
<210> 5
   <211> 2542
   <212> DNA
   <213> Bovine intestinal
<400> 5
   gaattcggca cgaggagacc cggcctcccc aggtcccatc ctgaccctcc gccatcacac 60
   agccatgcag tgggcctgtg tgctgctgct gctgggcctg tggctacagc tctccctcac 120
   cttcatccca gctgaggagg aagaccccgc cttctggaac cgccaggcag cccaggccct 180
   tgatgtagcc aagaagttgc agccgatcca gacagctgcc aagaatgtca tcctcttctt 240
   gggggatggg atgggggtgc ctacggtgac agccactcgg atcctaaagg ggcagatgaa 300
   tggtaagctg ggacctgaga cacccctggc catggaccag ttcccatacg tggctctgtc 360
   caagacatac aacgtggaca gacaggtgcc agacagcgca ggcactgcca ctgcctacct 420
   gtgtggggtc aagggcaact acaaaaccat tggtgtaagt gcagccgccc gctacaacca 480
   gtgcaacaca acaagtggca atgaggtcac gtctgtgatg aaccgggcca agaaagcagg 540
   aaagtcagtg ggagtggtga ccacctccag ggtgcagcat gcctccccag ccggtgctta 600
   tgcacacacg gtgaaccgaa actggtactc agatgccgac ctgcctgccg atgcacagac 660
   gtatggctgc caggacatcg ccacacaact ggtcaacaac atggatattg acgtgatcct 720
   gggtggaggc cgaatgtaca tgtttcctga ggggaccccg gatcctgaat acccatacga 780
   tgtcaatcag actggagtcc ggaaggacaa gcggaatctg gtgcaggagt ggcaggccaa 840
   gcaccaggga gcccagtatg tgtggaaccg cacggagctc cttcaggcag ccaatgaccc 900
   cagtgtaaca cacctcatgg gcctctttga gccggcagac atgaagtata atgttcagca 960
   agaccccacc aaggacccga ccctggagga gatgacggag gcggccctgc aagtgctgag 1020
   caggaacccc cagggcttct acctcttcgt ggagggaggc cgcattgacc acggtcacca 1080
   tgaaggcaaa gcttatatgg cactgactga tacagtcatg tttgacaatg ccatcgccaa 1140
   ggctaacgag ctcactagcg aactggacac gctgatcctt gccactgcag accactccca 1200
   tgtcttctct tttggtggct acacactgcg tgggacctcc attttcggtc tggcccccag 1260
   caaggcctca gacaacaagt cctacacctc catcctctat ggcaatggcc ctggctacgt 1320
   gcttggtggg ggcttaaggc ccgatgttaa tgacagcata agcgaggacc cctcgtaccg 1380
   gcagcaggcg gccgtgcccc tgtctagtga gtcccacggg ggcgaggacg tggcggtgtt 1440
   cgcgcgaggc ccgcaggcgc acctggtgca cggcgtgcag gaggagacct tcgtggcgca 1500
   cgtcatggcc tttgcgggct gcgtggagcc ctacaccgac tgcaatctgc cggccccctc 1560
   tggcctctcc gacgccgcgc acctggcggc cagcccgcct tcgctggcgc tgctggccgg 1620
   ggcgatgctg ctgctgctgg cgcctgcctt gtactgaccc ccaccaactc caggtcttgg 1680
   ggtttcctgc tttcctgcca aaaatctccc agcgcagacc ccaccagagc taccacctcg 1740
   gagtctccac cctgaagtcc tatcttagcg gccactcccg gatccccgac caggccccac 1800
   tagcagagct tcacctccca gaaatgaagg attcaccttc cagcaacgaa gaagcctcag 1860
   ctcacagccc ttcatggccc agcccatcca gaggctgagg ccctgatttc cctgtgacac 1920
   ccgtagacct actgcccgac cccaacttca gtggcttggg attttgtgtt ctgccacccc 1980
   taaccccagt aagggggctc ggaccatcca gactctcccc actgcccaca accccacctg 2040
   agaaccaggc tagcacggtc ccaaggttcc caggcccggc tagaacccac accatgcctt 2100
   tcaggagacc ctggggctcc ggggtttccg ggaggcgtgg ctttcttagg aggcgtggaa 2160
   actgaggagg cacggtttct gaggaggcgt gcgtcctggg gagctgtggc ttccggtcct 2220
   ccccatgccc tgtgggctcc tccctaacca aggagacggc caaggagacg tctggaacca 2280
   ggagcggcgg gggaaccttg cagagccctc agcaacccct cctaggaacc cagggtaccg 2340
   ttagagagag gagacagcga cacagaggag aggagacttg tcccaggtct ctcagctgct 2400
   atgaaggtgg ccccggtgcc ccttccaggc tgggagatcc caggagcagc gggggagctg 2460
   gtgggtgggg acacagcccc gccttcatgg gagggaggaa gcagccctca aataaactgt 2520
   tctaagtgtg aaaaaatcta ga 2542
<210> 6
   <211> 511
   <212> PRT
   <213> Bovine intestinal
<400> 6
   Phe Ile Pro Ala Glu Glu Glu Asp Pro Ala Phe Trp Asn Arg Gln Ala
   1               5                   10                  15
   Ala Gln Ala Leu Asp Val Ala Lys Lys Leu Gln Pro Ile Gln Thr Ala
               20                  25                  30
   Ala Lys Asn Val Ile Leu Phe Leu Gly Asp Gly Met Gly Val Pro Thr
           35                  40                  45
   Val Thr Ala Thr Arg Ile Leu Lys Gly Gln Met Asn Gly Lys Leu Gly
       50                  55                  60
   Pro Glu Thr Pro Leu Ala Met Asp Gln Phe Pro Tyr Val Ala Leu Ser
   65                  70                  75                  80
   Lys Thr Tyr Asn Val Asp Arg Gln Val Pro Asp Ser Ala Gly Thr Ala
                   85                  90                  95
   Thr Ala Tyr Leu Cys Gly Val Lys Gly Asn Tyr Lys Thr Ile Gly Val
               100                 105                 110
   Ser Ala Ala Ala Arg Tyr Asn Gln Cys Asn Thr Thr Ser Gly Asn Glu
           115                 120                 125
   Val Thr Ser Val Met Asn Arg Ala Lys Lys Ala Gly Lys Ser Val Gly
   130 135 140
   Val Val Thr Thr Ser Arg Val Gln His Ala Ser Pro Ala Gly Ala Tyr
   145 150 155 160
   Ala His Thr Val Asn Arg Asn Trp Tyr Ser Asp Ala Asp Leu Pro Ala
   165 170 175
   Asp Ala Gln Thr Tyr Gly Cys Gln Asp Ile Ala Thr Gln Leu Val Asn
   180 185 190
   Asn Met Asp Ile Asp Val Ile Leu Gly Gly Gly Arg Met Tyr Met Phe
   195 200 205
   Pro Glu Gly Thr Pro Asp Pro Glu Tyr Pro Tyr Asp Val Asn Gln Thr
   210 215 220
   Gly Val Arg Lys Asp Lys Arg Asn Leu Val Gln Glu Trp Gln Ala Lys
   225 230 235 240
   His Gln Gly Ala Gln Tyr Val Trp Asn Arg Thr Glu Leu Leu Gln Ala
   245 250 255
   Ala Asn Asp Pro Ser Val Thr His Leu Met Gly Leu Phe Glu Pro Ala
   260 265 270
   Asp Met Lys Tyr Asn Val Gln Gln Asp Pro Thr Lys Asp Pro Thr Leu
   275 280 285
   Glu Glu Met Thr Glu Ala Ala Leu Gln Val Leu Ser Arg Asn Pro Gln
   290 295 300
   Gly Phe Tyr Leu Phe Val Glu Gly Gly Arg Ile Asp His Gly His His
   305 310 315 320
   Glu Gly Lys Ala Tyr Met Ala Leu Thr Asp Thr Val Met Phe Asp Asn
   325 330 335
   Ala Ile Ala Lys Ala Asn Glu Leu Thr Ser Glu Leu Asp Thr Leu Ile
   340 345 350
   Leu Ala Thr Ala Asp His Ser His Val Phe Ser Phe Gly Gly Tyr Thr
   355 360 365
   Leu Arg Gly Thr Ser Ile Phe Gly Leu Ala Pro Ser Lys Ala Ser Asp
   370 375 380
   Asn Lys Ser Tyr Thr Ser Ile Leu Tyr Gly Asn Gly Pro Gly Tyr Val
   385                 390                 395                 400
   Leu Gly Gly Gly Leu Arg Pro Asp Val Asn Asp Ser Ile Ser Glu Asp
                   405                 410                 415
   Pro Ser Tyr Arg Gln Gln Ala Ala Val Pro Leu Ser Ser Glu Ser His
               420                 425                 430
   Gly Gly Glu Asp Val Ala Val Phe Ala Arg Gly Pro Gln Ala His Leu
           435                 440                 445
   Val His Gly Val Gln Glu Glu Thr Phe Val Ala His Val Met Ala Phe
       450                 455                 460
   Ala Gly Cys Val Glu Pro Tyr Thr Asp Cys Asn Leu Pro Ala Pro Ser
   465                 470                 475                 480
   Gly Leu Ser Asp Ala Ala His Leu Ala Ala Ser Pro Pro Ser Leu Ala
                   485                 490                 495
   Leu Leu Ala Gly Ala Met Leu Leu Leu Leu Ala Pro Ala Leu Tyr
               500                 505                 510
<210> 7
   <211> 18
   <212> DNA
   <213> artificial Sequence
<220>
   <223> Description of Artificial Sequence: Artificial
<400> 7
   gccaagaatg tcatcctc                                                18
<210> 8
   <211> 18
   <212> DNA
   <213> Artificial Sequence
<220>
   <223> Description of Artificial Sequence: Artificial
<400> 8
   gaggatgaca ttcttggc                                                18
<210> 9
   <211> 17
   <212> DNA
   <213> Artificial Sequence
<220>
   <223> Description of Artificial Sequence: Artificiall
<400> 9
   ggtgtaagtg cagccgc 17
<210> 10
   <211> 17
   <212> DNA
   <213> Artificial Sequence
<220>
   <223> Description of Artificial Sequence: Artificial
<400> 10
   gcggctgcac ttagacc 17
<210> 11
   <211> 17
   <212> DNA
   <213> Artificial Sequence
<220>
   <223> Description of Artificial Sequence: Artificial
<400> 11
   aatgtacatg tttcctg 17
<210> 12
   <211> 17
   <212> DNA
   <213> Artificial Sequence
<220>
   <223> Description of Artificial Sequence: Artificial
<400> 12
   caggaaacat gtacatt 17
<210> 13
   <211> 18
   <212> DNA
   <213> Artificial Sequence
<220>
   <223> Description of Artificial Sequence: Artificial
<400> 13
   ccagggcttc tacctctt 18
<210> 14
   <211> 18
   <212> DNA
   <213> Artificial Sequence
<220>
   <223> Description of Artificial Sequence: Artificial
<400> 14
   aagaggtaga agccctgg 18
<210> 15
   <211> 18
   <212> DNA
   <213> Artificial Sequence
<220>
   <223> Description of Artificial Sequence: Artificial
<400> 15
   accagagcta ccacctcg 18
<210> 16
   <211> 18
   <212> DNA
   <213> Artificial Sequence
<220>
   <223> Description of Artificial Sequence: Artificial
<400> 16
   aagcaggaaa ccccaaga 18
<210> 17
   <211> 18
   <212> DNA
   <213> Artificial Sequence
<220>
   <223> Description of Artificial Sequence: Artificial
<400> 17
   cttcagtggc ttgggatt 18
<210> 18
   <211> 18
   <212> DNA
   <213> Artificial Sequence
<220>
   <223> Description of Artificial Sequence: Artificial
<400> 18
   aatcccaagc cactgaag 18
<210> 19
   <211> 17
   <212> DNA
   <213> Artificial Sequence
<220>
   <223> Description of Artificial Sequence: Artificial
<400> 19
   cgaggtcgac ggtatcg 17
<210> 20
   <211> 30
   <212> DNA
   <213> Artificial Secuence
<220>
   <223> Description of Artificial Sequence: Artificial
<400> 20
   gcaggtctct cagctgggat gagggtgagg 30
<210> 21
   <211> 30
   <212> DNA
   <213> Artificial Sequence
<220>
   <223> Description of Artificial Sequence: Artificial
<400> 21
   gcaggtctca gctgaggagg aaaaccccgc 30
<210> 22
   <211> 27
   <212> DNA
   <213> Artificial Sequence
<220>
   <223> Description of Artificial Sequence: Artificial
<400> 22
   gcaggtctct gttgtgtcgc actggtt 27
<210> 23
   <211> 27
   <212> DNA
   <213> Artificial Sequence
<220>
   <223> Description of Artificial Sequence: Artificial
<400> 23
   ggtctctttc ttggcccggt tgatcac 27
<210> 24
   <211> 27
   <212> DNA
   <213> Artificial Sequence
<220>
   <223> Description of Artificial Sequence: Artificial
<400> 24
   ggtctcaaga aagcagggaa ggccgtc 27
<210> 25
   <211> 28
   <212> DNA
   <213> Artificial Sequence
<220>
   <223> Description of Artificial Sequence: Artificial
<400> 25
   ggtctcgtgc atcagcaggc aggtcggc 28
<210> 26
   <211> 29
   <212> DNA
   <213> Artificial Sequence
<220>
   <223> Description of Artificial Sequence: Artificial
<400> 26
   ggtctcatgc acagaagaat ggctgccag 29
<210> 27
   <211> 30
   <212> DNA
   <213> Artificial Sequence
<220>
   <223> Description of Artificial Sequence: Artificial
<400> 27
   ggtctcaaac atgtacattc ggcctccacc 30
<210> 28
   <211> 27
   <212> DNA
   <213> Artificial Sequence
<220>
   <223> Description of Artificial Sequence: Artificial
<400> 28
   gtctccatgt ttcctgaggg gacccca 27
<210> 29
   <211> 27
   <212> DNA
   <213> Artificial Sequence
<220>
   <223> Description of Artificial Sequence: Artificial
<400> 29
   ggtctcctgc cattcctgca ccaggtt 27
<210> 30
   <211> 27
   <212> DNA
   <213> Artificial Sequence
<220>
   <223> Description of Artificial Sequence: Artificial
<400> 30
   ggtctctggc aggccaagca ccaggga 27
<210> 31
   <211> 27
   <212> DNA
   <213> Artificial Sequence
<220>
   <223> Description of Artificial Sequence: Artificial
<400> 31
   ggtctccagg gtcgggtcct tggtgtg 27
<210> 32
   <211> 25
   <212> DNA
   <213> Artifcial Sequence
<220>
   <223> Description of Artificial Sequence: Artificial
<400> 32
   ggtctcgacc ctggcggaga tgacg 25
<210> 33
   <211> 24
   <212> DNA
   <213> Artificial Sequence
<220>
   <223> Description of Artificial Sequence: Artificial
<400> 33
   ggtctcctca gtcagtgcca tata 24
<210> 34
   <211> 27
   <212> DNA
   <213> Artificial Sequence
<220>
   <223> Description of Artificial Sequence: Artificial
<400> 34
   ggtctcactg aggcgatcat gtttgac 27
<210> 35
   <211> 23
   <212> DNA
   <213> Artificial Sequence
<220>
   <223> Description of Artificial Sequence: Artificial
<400> 35
   tgcaccaggt gcgcctgcgg gcc 23
<210> 36
   <211> 27
   <212> DNA
   <213> Artificial Sequence
<220>
   <223> Description of Artificial Sequence: Artificial
<400> 36
   gccgcacagc tggtctacaa catggat 27
<210> 37
   <211> 24
   <212> DNA
   <213> Artificial Sequence
<220>
   <223> Description of Artificial Sequence: Artificial
<400> 37
   gctgtctaag gccttgccgg gggc 24
<210> 38
   <211> 27
   <212> DNA
   <213> Artificial Sequence
<220>
   <223> Description of Artificial Sequence: Artificial
<400> 38
   gccgcacagc tggtctacaa catggat 27
<210> 39
   <211> 25
   <212> DNA
   <213> Artificial Sequence
<220>
   <223> Description of Artificial Sequence: Artificial
<400> 39
   gggggtctcg cttgctgcca ttaac 25
<210> 40
   <211> 30
   <212> DNA
   <213> Artificial Sequence
<220>
   <223> Description of Artificial Sequence: Artificial
<400> 40
   gttaatggtc tcacaagcga ggaaccctcg 30
<210> 41
   <211> 27
   <212> DNA
   <213> Artificial Sequence
<220>
   <223> Description of Artificial Sequence: Artificial
<400> 41
   cccgtgggtc tcgctagcca gcggcac 27
<210> 42
   <211> 30
   <212> DNA
   <213> Artificial Sequence
<220>
   <223> Description of Artificial Sequence: Artificial
<400> 42
   gttaatggtc tcacaagcga ggaaccctcg 30
<210> 43
   <211> 30
   <212> DNA
   <213> Artificial Sequence
<220>
   <223> Description of Artificial Sequence: Artificial
<400> 43
   gatgctggtc tcggtggagg gggctggcag 30
<210> 44
   <211> 30
   <212> DNA
   <213> Artificial Sequence
<220>
   <223> Description of Artificial Sequence: Artificial
<400> 44
   ctgccaggtc tcaccaccgc caccagcatc 30
<210> 45
   <211> 24
   <212> DNA
   <213> Artificial Sequence
<220>
   <223> Description of Artificial Sequence: Artificial
<400> 45
   catacgattt aggtgacact atag 24
<210> 46
   <211> 27
   <212> DNA
   <213> Artificial Sequence
<220>
   <223> Description of Artificial Sequence: Artificial
<400> 46
   ggtctctggc aggccaagca ccaggga 27
<210> 47
   <211> 24
   <212> DNA
   <213> Artificial Sequence
<220>
   <223> Description of Artificial Sequence: Artificial
<400> 47
   gtagaagccc cgggggttcc tgct 24
<210> 48
   <211> 24
   <212> DNA
   <213> Artificial Sequence
<220>
   <223> Description of Artificial Sequence: Artificial
<400> 48
   agcaggaacc cccggggctt ctac 24
<210> 49
   <211> 27
   <212> DNA
   <213> Artificial Sequence
<220>
   <223> Description of Artificial Sequence: Artificial
<400> 49
   tgccatataa gctttgccgt catggtg 27
<210> 50
   <211> 27
   <212> DNA
   <213> Artificial Sequence
<220>
   <223> Description of Artificial Sequence: Artificial
<400> 50
   ggtctctttc ttggcccggt tcatcac 27
<210> 51
   <211> 27
   <212> DNA
   <213> Artificial Sequence
<220>
   <223> Description of Artificial Sequence: Artificial
<400> 51
   tggtcaccac tcccacggac ttccctg 27
<210> 52
   <211> 30
   <212> DNA
   <213> Artificial Sequence
<220>
   <223> Description of Artificial Sequence: Artificial
<400> 52
   ggtctcaaac atgtattttc ggcctccacc 30
<210> 53
   <211> 30
   <212> DNA
   <213> Artificial Sequence
<220>
   <223> Description of Artificial Sequence: Artificial
<400> 53
   ggtctcatgt ttcctgtggg gaccccagac 30
<210> 54
   <211> 27
   <212> DNA
   <213> Artificial Sequence
<220>
   <223> Description of Artificial Sequence: Artificial
<400> 54
   ggtctcctgc catgcctgca ccaggtt 27